(19) Europäisches Patentamt
European Patent Office
Office européen des brevets

(11) Veröffentlichungsnummer: **0 224 086**
**B1**

(12) **EUROPÄISCHE PATENTSCHRIFT**

(45) Veröffentlichungstag der Patentschrift: **24.10.90**

(21) Anmeldenummer: **86115358.3**

(22) Anmeldetag: **05.11.86**

(51) Int. Cl.[5]: **C 07 D 215/26,** A 61 K 31/47
// C07D215/28, C07D215/36,
C07D215/40, C07D215/60,
C07D217/02, C07D277/64,
C07D277/76, A61K31/425

(54) **Substituierte Benzylether.**

(30) Priorität: **16.11.85 DE 3540743**
**22.05.86 DE 3617183**

(43) Veröffentlichungstag der Anmeldung:
**03.06.87 Patentblatt 87/23**

(45) Bekanntmachung des Hinweises auf die Patenterteilung:
**24.10.90 Patentblatt 90/43**

(84) Benannte Vertragsstaaten:
**AT BE CH DE ES FR GB IT LI NL SE**

(56) Entgegenhaltungen:
**EP-A-0 079 168**
**EP-A-0 110 405**
**EP-A-0 113 587**
**EP-A-0 181 568**
**FR-A-2 253 511**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Mohrs, Klaus-Helmut, Dr.**
**Claudiusweg 9**
**D-5600 Wuppertal 1 (DE)**
Erfinder: **Fruchtmann, Romanis, Dr.**
**Konrad-Adenauer-Ufer 79**
**D-5000 Köln 1 (DE)**
Erfinder: **Kohlsdorfer, Christian, Dr.**
**Franz-Stryck-Strasse 16**
**D-5042 Erftstadt (DE)**

(56) Entgegenhaltungen:

**CHEMICAL ABSTRACTS, Band 68, Nr. 17, 22. April 1968, Seite 7594, Zusammenfassung Nr. 78579u, Columbus, Ohio, US; S.C. SMITH et al.: "Hydroxymethylation of the benzene ring. III. Synthesis of the D- and L-isomers of p-(hydroxymethyl)phenylalanine and the biochemical properties of the L-isomer"**

**CHEMICAL ABSTRACTS, Band 78, Nr. 1, 8. Januar 1973, Seite 3906, Zusammenfassung nr. 3907x, Columbus, Ohio, US; E. GROCHOWSKI et al.: "Electrophilic reactions of the cyano group. VIII. Intramolecular cyclization of 2-hydroxy nitriles"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 224 086 B1

**Beschreibung**

Die Erfindung betrifft neue substituierte Benzylether, -thioether und -amine, Verfahren zu ihrer Herstellung und ihre Verwendung in Arzneimitteln.

Aus EP—OS 110 405 ist bekannt, daß Benzyl-2-(1-hydroxyalkyl)phenylether sowie Benzyl-3-(1-hydroxyalkyl)phenylether antiallergische Wirkung besitzen.

In EP 181 568, ebenso wie in EP 110 405 werden unter anderem Chinolinylmethoxy-phenyl-Verbindungen mit lipoxygenasehemmender Wirkung beschrieben, wobei der Methoxyphenylrest am heterocyclischen Teil des Chinolinringes gebunden ist.

Die vorliegende Erfindung betrifft neue substituierte Benzylverbindungen der allgemeinen Formel (I)

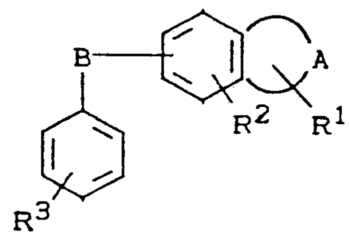

(I)

in welcher

$R^1$, $R^2$ gleich oder verschieden sind und für Wasserstoff, $C_1$ bis $C_8$-Alkyl, $C_2$ bis $C_6$-Alkenyl, Cyclopentyl, Cyclohexyl, $C_1$ bis $C_6$-Alkoxy, $C_1$ bis $C_6$-Alkylthio, Halogen $C_1$ bis $C_6$-Alkyl, Halogen $C_1$ bis $C_6$-Alkoxy Halogen $C_1$ bis $C_6$-Alkylthio, Benzyloxy, Benzylthio, Fluor, Chlor, Brom, Nitro, Cyano, Hydroxy oder für eine Gruppe der Formel

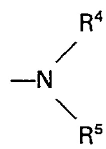

stehen,

wobei

$R^4$ und $R^5$ gleich oder verschieden sind und für Wasserstoff, $C_1$ bis $C_8$-Alkyl, $C_2$ bis $C_6$-Alkenyl, Cyclopentyl, Cyclohexyl, Benzyl, Phenyl, Benzoyl oder Acetyl stehen,

$R^3$ für eine Gruppe der Formel

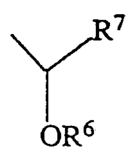

,

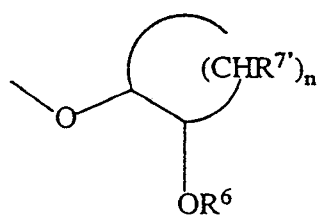

oder

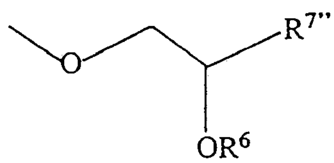

steht

worin

$R^6$ für Wasserstoff, Benzoyl, oder $C_1$ bis $C_6$-Alkyl-CO- steht,

$R^7$, $R^{7'}$,$R^{7''}$ gleich oder verschieden sind und für Wasserstoff oder $C_1$ bis $C_8$-Alkyl stehen und

n für eine Zahl von 3 bis 8 steht,

B für $—CH_2—X—$ steht, wobei

X für O, S oder $NR^7$ steht und wobei

$R^7$ die oben genannte Bedeutung hat, und die Gruppe der Formel

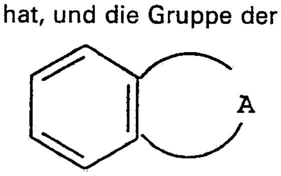

für Chinolin, Isochinolin, Cinnolin, Chinolin-N-oxid, Chinoxalin, Phthalazin, Chinolon, Isochinolon,

Cinnolinon, Chinazolindion, Chinoxazolinon, Chinoxalindion, Phthalazindion, Indol, Indolon, Isoindol, Isoindolon, Benzofuran, Benzothiophen, Benzimidazol, Benzoxazol, Benzothiazol, Indazol, Indoxyl steht.

Im Vergleich zu den bekannten Benzylethern haben die erfindungsgemäßen substituierten Benzylether überraschenderweise eine höhere pharmakologische Wirkung.

Halogen steht im allgemeinen für Fluor, Chlor, Brom oder Iod, bevorzugt für Fluor, Chlor oder Brom. Besonders bevorzugt steht Halogen für Fluor oder Chlor.

Es werden Verbindungen der allgemeinen Formel (I) bevorzugt, in denen

$R^1$, $R^2$ gleich oder verschieden sind und für Wasserstoff, Methyl, Ethyl Propyl, Isopropyl, Allyl, Methoxy, Ethoxy, Methylthio, Ethylthio, Trifluormethylthio, Fluor, Chlor Brom, Nitro, Cyano, Hydroxy oder für eine Gruppe der Formel

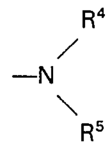 stehen,

wobei

$R^4$ und $R^5$ gleich oder verschieden sind und für Wasserstoff, Methyl, Ethyl, Propyl, Isopropyl, Allyl, Benzyl, Phenyl oder Acetyl stehen,

$R^3$ für einen Rest der Formel

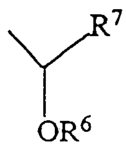 ,

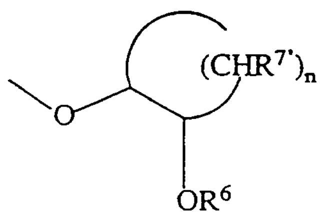 oder

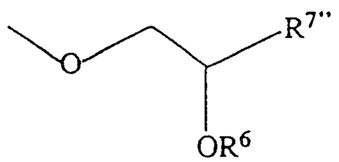 steht

wobei

$R^6$ für Wasserstoff, Benzoyl, Acetyl, Ethylcarbonyl oder Propylcarbonyl steht,

$R^7$, $R^{7'}$, $R^{7''}$ gleich oder verschieden sind und für Wasserstoff oder für eine gerade oder verzweigte Alkylkette mit bis zu 8 C-Atomen stehen und

n für eine Zahl von 3 bis 4 steht,

B für $—CH_2—X—$ steht, wobei

X für O, S oder $NR^7$ steht und wobei

$R^7$ die oben genannte Bedeutung hat, und die Gruppe der Formel

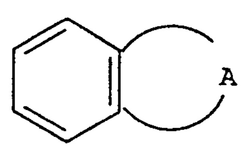

für Chinolin, Chinolin-N-oxid, Isochinolin, Isochinolin-N-oxid, Cinnolin, Chinazolin, Chinoxalin, Phthalazin, Chinolon, Isochinolon, Indol, Indolon, Isoindol, Benzofuran, Benzothiophen, Benzimidazol, Benzothiazol, Benzoxazol oder Indazol steht.

Besonders bevorzugte Verbindungen der allgemeinen Formel (I), sind solche, in denen

$R^1$, $R^2$ gleich oder verschieden sind und für Wasserstoff, Methyl, Ethyl, Fluor, Chlor, Brom und Nitro stehen,

$R^3$ für eine Gruppe der Formel

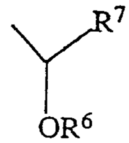 , 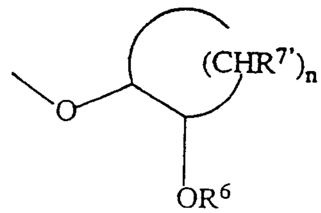 oder

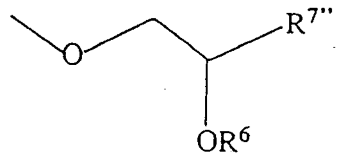 steht

worin

$R^6$ Wasserstoff, Acetyl, Ethylcarbonyl oder Benzoyl bedeutet, $R^7$, $R^{7'}$, $R^{7''}$ gleich oder verschieden sind und Wasserstoff oder eine geradkettige oder verzweigte Alkylkette mit bis zu 6 C-Atomen bedeutet,

n für eine Zahl 3 oder 4 steht,

B für $—CH_2—O—$, $—CH_2S—$ oder $—CH_2NH—$ steht, und die Gruppe der Formel

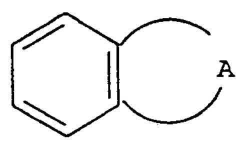

für Chinolin, Chinolin-N-oxid, Isochinolin, oder Chonlon steht.

Beispeilsweise seien folgende Wirkstoffe im einzelnen genannt:

Essigsäure-1-[3-(chinolin-8-yloxymethyl)phenyl]pentylester
Essigsäure-1-[3-(chinolin-8-yloxymethyl)phenyl]hexylester
Essigsäure-1-[3-(chinolin-8-yloxymethyl)phenyl]heptylester
Essigsäure-1-[3-(5,7-dichlorchinolin-8-yloxymethyl)phenyl]heptylester
Essigsäure-1-[2-(5,7-dichlorchinolin-8-yloxymethyl)phenyl]pentylester
Essigsäure-2-[3-(chinolin-8-yloxymethyl)phenoxy]cyclohexylester
Essigsäure-1-[2-(2-(1H)-chinolon-8-yloxymethyl)phenoxymethyl]propylester
Essigsäure-2-[3-(2-(1H)-chinolon-8-yloxymethyl)phenoxy]cyclohexylester (trans-Form)
Essigsäure-1-[3-(2-(1H)-chinolon-8-yloxymethyl)phenyl]hexylester
Essigsäure-1-[3-(2-(1H)-chinolin-8-yloxymethyl)phenyl]heptylester
Essigsäure-1-[3-(2-(1H)-chinolon-8-yloxymethyl)-phenoxymethyl)propylester
Essigsäure-1-[2-(2-(1H)-chinolon-8-yloxymethyl)phenyl]hexylester
Essigsäure-1-[2-(2-chinolin-8-yloxymethyl)phenyl]heptylester
Essigsäure-1-[2-(chinolin-N-oxid-8-yloxymethyl)phenyl]hexylester
Essigsäure-1-[3-(chinolin-8-yloxymethyl)phenyl]hexylester
Essigsäure-1-[4-(chinolin-8-yloxymehtyl)phenyl]pentylester
Essigsäure-1-[3-(isochinolin-5-yloxymethyl)phenyl]pentylester
Essigsäure-1-[3-(chinolin-8-ylaminomethyl)phenyl]pentylester
Essigsäure-1-[3-(chinolin-8-ylthiomethyl)phenyl]pentylester
Essigsäure-1-[3-(chinolin-8-yloxymethyl)phenoxymethyl]pentylester
Essigsäure-1-[3-(chinolin-8-yloxymethyl)phenoxymethyl]propylester
Essigsäure-1-[2-(chinolin-8-yloxymethyl)phenoxymethyl]propylester
Essigsäure-1-[3-(chinolin-8-yloxymethyl)phenoxymethyl]-2,2-dimethyl-propylester
Essigsäure-1-[3-(5,7-dichlorochinolin-8-yloxymethyl)phenyl]pentylester
1-[3-(Chinolin-8-yloxymethyl)phenoxymethyl]-2,2-dimethylpropanol
1-[3-(Chinolin-8-yloxymethyl)phenoxymethyl]pentanol
1-[3-(Isochinolin-5-yloxymethyl)phenyl]pentanol
1-[3-(Chinolin-8-ylaminomethyl)phenyl]pentanol
1-[3-(2-Methylchinolin-8-yloxymethyl)phenyl]hexanol
1-[2-(1-Oxochinolin-8-yloxymethyl)phenyl]hexanol
1-[2-(Chinolin-8-yloxymethyl)phenoxymethyl]propanol
1-[3-(Chinolin-8-yloxymethyl)phenoxymethyl]propanol
1-[3-(Chinolin-8-ylthiomethyl)phenyl]pentanol
1-[3-(Chinolin-8-yloxymethyl)phenyl]heptanol

1-[3-(Chinolin-8-yloxymethyl)phenyl)hexanol
1-[3-(2-Chinolin-8-yloxymethyl)phenyl]heptanol
1-[3-(2-Chinolin-8-yloxymethyl)phenyl]hexanol
2-[3-(2-(1H)-Chinolon-8-yloxymethyl)phenoxy]cyclohexanol (trans-Form)
1-[2-(Chinolin-8-yloxymethyl)phenyl]pentanol
1-[3-(5,7-Dichlorchinolin-8-yloxymethyl)phenyl]heptanol
1-[3-(2-(1H)-Chinolon-8-yloxymethyl)phenoxymethyl]propanol
1-[2-(Chinolin-8-yloxymethyl)phenyl]hexanol
1-[3-(Chinolin-8-yloxymethyl)phenyl]pentanol
1-[2-(2-(1H)-Chinolon-8-yloxymethyl)phenyl]hexanol
1-[2-(2-(1H)-Chinolon-8-yloxymethyl)phenoxymethyl]propanol
1-[2-(2-(1H)-Chinolon-8-yloxymethyl)phenyl]heptanol
Essigsäure-1-[3-(chinolin-8-yloxymethyl)phenyl]-3-methylbutylester
Propionsäure-1-[3-(chinolin-8-yloxymethyl)phenyl]hexylester
Essigsäure-1-[3-(4-methylchinolin-8-yloxymethyl)phenyl]pentylester
Essigsäure-1-[3-(4-chlorchinolin-8-yloxymethyl)phenyl]pentylester
Essigsäure-1-[3-(6-methylchinolin-8-yloxymethyl)phenyl]pentylester
Essigsäure-1-[3-(6-nitrochinolin-8-yloxymethyl)phenyl]pentylester
Essigsäure-1-[3-(2-ethylsulfonylbenzothiazol-7-yl-aminomethyl)phenyl]hexylester
1-[4-Chinolin-8-yloxymethyl)phenyl]pentanol
1-[3-(Chinolin-8-yloxymethyl)phenyl]-3-methylbutanol
1-[3-(2-Ethylsulfonylbenzothiazol-7-yloxymethyl)phenyl]hexanol
1-[3-(4-Chlorchinolin-8-yloxymethyl)phenyl]pentanol
1-[3-(4-Methylchinolin-8-yloxymethyl)phenyl]pentanol
1-[3-(6-Methylchinolin-8-yloxymethyl)phenyl]pentanol
8-[4-(1-Methoxypentyl)benzyl]oxychinolin
Benzoesäure-1-[3-(chinolin-8-yloxymethyl)phenyl]pentylester

Es wurde weiterhin ein Verfahren zur Herstellung von substituierten Benzylethern der Formel

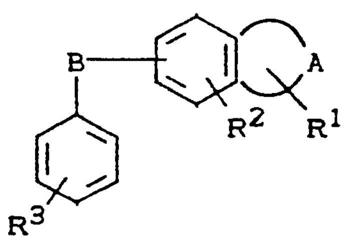

(I),

in welcher
$R^1$, $R^2$, $R^3$, B und die Gruppe der Formel

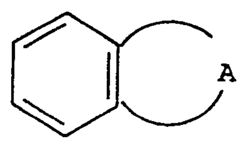

die oben angegebene Bedeutung haben, gefunden, das dadurch gekennzeichnet ist, daß man
Halogenide der allgemeinen Formel (II)

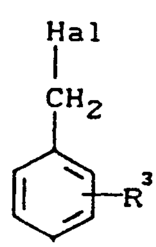

(II),

worin
$R^3$ die angegebene Bedeutung hat, und
Hal für Chlor, Brom oder Iod steht, mit Verbindungen der allgemeinen Formel (III),

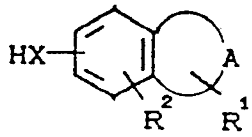

(III),

in welcher

$R^1$, $R^2$, X und die Gruppe der Formel

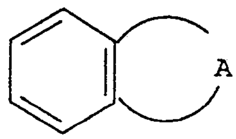

die angegebene Beduetung haben, in inerten organischen Lösungsmitteln gegebenenfalls in Anwesenheit einer Base umsetzt, dann gegebenenfalls vorhandene Acylgruppen abspaltet, und dann gegebenenfalls die Hydroxyverbindungen alkyliert.

Das erfindungsgemäße Verfahren kann beispielsweise durch das folgende Formelschema a) erläutert werden:

a)

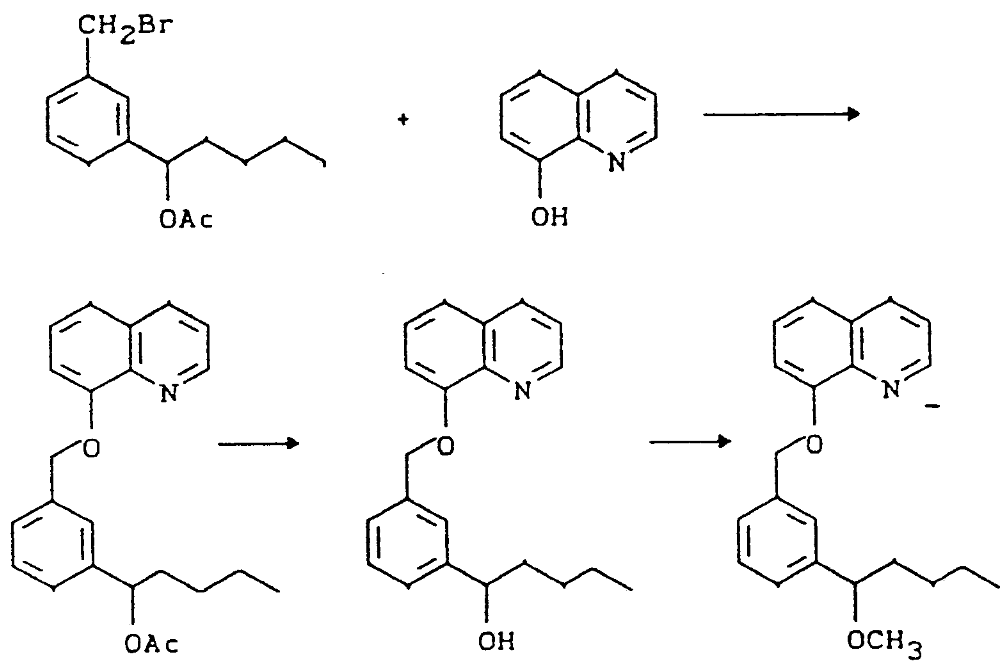

Lösungsmittel für das erfindungsgemäße Verfahren können inerte organische Lösungsmittel sein, die sich unter den Reaktionsbedingungen nicht verändern. Hierzu gehören vorzugsweise Alkohole wie beispielsweise Methanol, Ethanol, Propanol oder Isopropanol, Ether wie beispielsweise Dioxan, Tetrahydrofuran oder Diethylether, Halogenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösungsmittel einzusetzen.

Als Basen für das erfindungsgemäße Verfahren können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder organische Amine (Trialkyl($C_1$—$C_6$)amine) wie Triethylamin und Heterocyclen wie Pyridin, Methylpiperidin, Piperidin oder Morpholin.

Es ist auch möglich als Basen Alkalimetalle, wie Natrium, und deren hydride, wie Natriumhydrid, einzusetzen.

Die Herstellung der erfindungsgemäßen Verbindungen erfolgt im allgemeinen in einem Temperaturbereich von 0°C bis 150°C, bevorzugt von 10°C bis 100°C.

Das erfindungsgemäße Verfahren wird im allgemeinen bei Normaldruck durchgeführt. Es ist aber auch

möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Die Acylgruppe kann beispielsweise durch Zusatz von Basen abgespaltet werden. Als Basen seien beispielsweise Natriumhydroxid, Kaliumhydroxid, Bariumhydroxid, Natriumcarbonat oder Kaliumcarbonat genannt. Man setzt im allgemeinen 1 bis 5 mol, bevorzugt 2 bis 4 mol der Base bezogen auf 1 mol Acylverbindung ein.

Die Alkylierung der Hydroxyverbindungen (Formel I mit $R^6$=H) erfolgt im allgemeinen in inerten Lösungsmitteln, gegebenenfalls in Anwesenheit einer Base, mit üblichen Alkylierungsmitteln wie Alkylhalogeniden, z.B. $C_1$—$C_4$-Alkylchloride, -bromide oder -iodide, Dialkylsulfaten, z.B. Dimethylsulfat, oder Diazoalkanen, z.B. Diazomethan, Diazoethan oder Diazopropan.

Als Lösungsmittel eignen sich hierbei, je nach Art des Alkylierungsmittels alle inerten organischen Lösungsmittel. Hierzu gehören bevorzugt Ether wie diethylether, Dioxan oder Tetrahydrofuran, oder Kohlenwasserstoffe wie Benzol, Toluol oder Xylol, oder Dimethylformamid oder Hexamethylphosphorsäuretriamid, oder Gemische der genannten Lösungsmittel.

Als Basen eingen sich die üblichen basischen Verbindungen, Hierzu gehören bevorzugt Alkalihydride wie Natriumhydrid, Alkaliamide wie Natriumamid oder lithiumdiisopropylamid, Alkalialkoholate wie Natriummethanolat, Natriumethanolat, Kaliummethanolat, Kaliumethanolat oder Kalium-tertbutylat, oder organische Amine wie Trialkylamine z.B. Triethylamin, oder lithiumorganische Verbindungen wie Butyllithium oder Phenyllithium.

Das erfindungsgemäße Verfahren kann beispielsweise wie folgt durchgeführt werden:

Die OH—, SH— oder NH-acide-Ausgangsverbindung, ein Verdünnungsmittel und gegebenenfalls eine Base werden gemischt und mit dem Halogenid gegebenenfalls in einem Verdünnungsmittel versetzt. Die Mischung kann gegebenenfalls erwärmt werden. Die aufarbeitung erfolgt in an sich bekannter Weise.

Die Ausgangsverbindungen der allgemeinen Formel (III) sind bekannt oder können nach bakannten Methoden hergestellt werden (A. R. Katrityky, C. W. Reeds, Comprehensive Heterocyclic Chemistry Band 1—8, Pergamon Press).

Die als Ausgangsverbindungen verwendeten Benzylhalogenide der Formel (II) sind teilweise neu. Sie können hergestellt werden indem man Tolylverbindungen der allgemeinen Formel (VI)

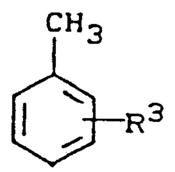

(VI),

in welcher

$R^3$ die angegebene Bedeutung hat, in geeigneten Lösungsmitteln mit Halogenierungsmitteln, gegebenenfalls in Anwesenheit von Radikalbildnern, unsetzt.

Die Reaktion läßt sich durch folgendes Schema Verdeutlichen:

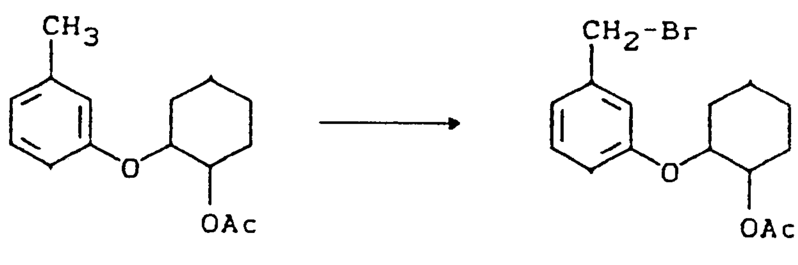

Als Lösungsmittel eigenen sich alle inerten organischen Lösungsmittel, die sich unter den Reaktionsbedingungen nicht verändern.

Hierzu gehören bevorzugt Halogenkohlenwasserstoffe zie zum Beispiel Dichlormethan, Trichlormethan oder Tetrachlormethan, Kohlenwasserstoffe wie Benzol, Toluol, Xylol, oder Eisessig.

Als Halogenierungsmittel können die allgemein üblichen Halogenierungsmittel verwendet werden. Bevorzugt sind Chlor, Brom, N-Chlorsuccinimid (NCS) oder N-Bromsuccinimid (NBS), gegebenenfalls in Anwesenheit von Radikalbildnern wie Azobisisobuttersäurentril (AIBN), Benzoylperoxid oder Licht. Besonders bevorzugt ist die Bromierung mit NBS und AIBN in Tetrachlormethan.

Die Reaktionstemperaturen können im allgemeinen in einem großen Bereich variiert werden. Bevorzugt arbeitet man in einem Bereich von −10°C bis 100°C, besonders bevorzugt von 0°C bis 80°C.

Die Umsetzung kann bei Normaldruck, aber auch bei erhöhtem oder erniedrigtem Druck durchgeführt werden. Im allgemeinen arbeitet man bei normalem Druck.

Das Mengenverhältnis der Reaktanden ist im allgemeinen beliebig. Bevorzugt arbeitet man jedoch mit einer Menge von 1 bis 5 mol, besonders bevorzugt mit 1 mol des Halogenierungsmittels bezogen auf 1 mol der Tolylverbindungen.

Die als Ausgangsverbindung verwendeten Tolylverbindungen sind bekannt oder können nach bekannten Methoden gemäß folgendem allgemeinen Schema hergestellt werden:

a. für $R^3$ = 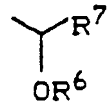

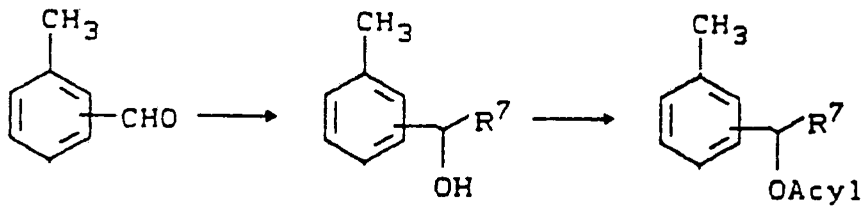

(VII)

Danach werden die entsprechenden Tolylaldehyde (VII) mit metallorganischen Verbindungen, bevorzugt Lithium-organischen Verbindungen oder Grignard Reagenzien nach bekannten Methoden zu den entsprechenden Alkoholen umgesetzt und anschließend an der Hydroxygruppe nach üblichen Methoden acyliert. Die Verfahren werden beispielweise von U. Schöllkopf in Houben-Weyls "Methoden der organischen Chemie" XIII/1, S. 175 ff; von K. Nützel in Houben-Weyls "Methoden der organischen Chemie" XIII/2a, S. 285 ff; sowie von E. Schumann in Houben-Weyls "Methoden der organischen Chemie" VI/1b, S. 823 ff, beschrieben.

b.

für $R^3$ = 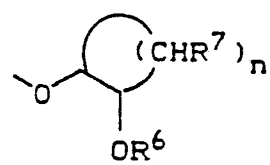 oder 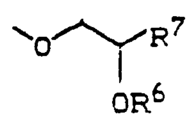

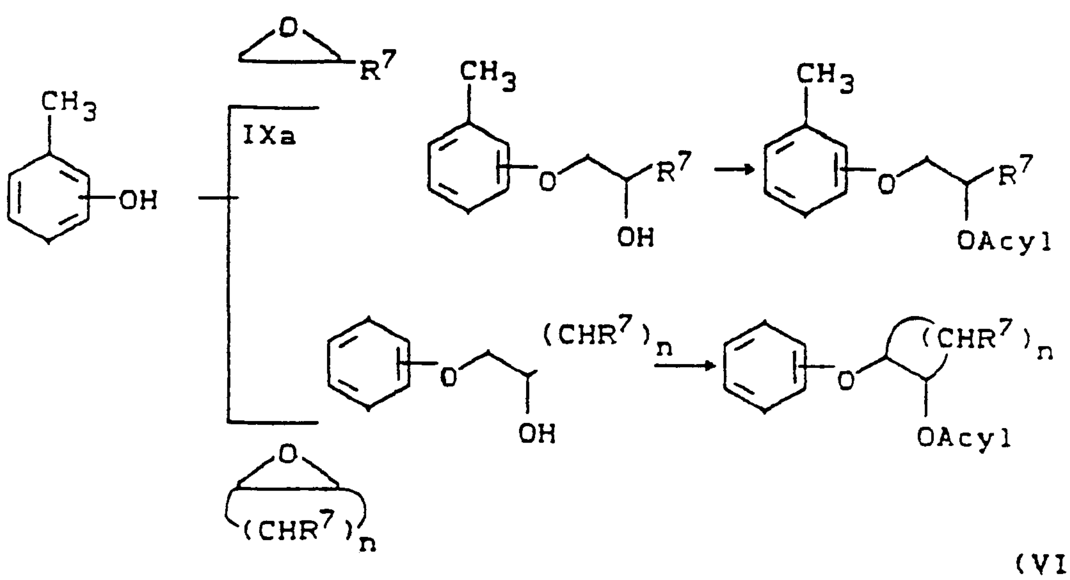

IXa

IXb

(VIII)

Danach werden die entsprechenden Kresole (VIII) mit Epoxiden IXa, b zu den entsprechenden Hydroxyethern nach bekannten Methoden umgesetzt und anschließend die Hydroxygruppe nach

bekannten Methoden acyliert. Diese Verfahren werden zum Beispiel von G. Dittus in Houben-Weyls "Methoden der organischen Chemie" VI/3, S. 456 ff., sowie von E. Schaumann in Houben-Weyls "Methoden der organischen Chemie" VI/1b, S. 823 ff., beschrieben.

Die als Ausgangsstoffe verwendeten Tolylaldehyde und Kresole sind bekannt: Beilstein's Handbuch der organischen Chemie, *7*, 295—297, und *6*, 349, 373 und 389.

Die erfindungsgemäßen Benzylverbindungen können als Wirkstoffe in Arzneimitteln eingesetzt werden. Die Stoffe wirken als Hemmer (Stimulatoren) von enzymatischen Reaktionen im Rahmen des Arachidonsäurestoffwechsels, insbesondere der Lipoxygenase.

Sie sind somit bevorzugt zu Behandlung und Verhütung von Erkrankungen der Atemwege wie Allergien/Asthma, Bronchitis, Emphysen, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, Metastasen und zur Cytoprotection im Gastrointestinaltrakt geeignet.

Die neuen Wirkstoffe können in an sich bekannter Weise unter Verwendung inerter nichttoxischer, pharmazeutischer geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen, Hierbei soll die therapeutisch wirksame Verbindung jeweils in einer Konzentration von etwa 0,5 bis 90 Gew.-%, bevorzugt von 10 bis 70 Gew.-%, in der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfstoffe seien beispielsweise aufgeführt: Wasser, nichttoxische organische Lösungsmittel wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silkate), Zucker (z.B. Rohr-, Milch- und Traubenzucker), Emulgiermittel (z.B. Polyoxyethylen-Fettsäureester, Polyoxyethylen-Fettalkohole-Ether, alkylsulfonate und Arylsulfonate), Dispergiermittel (z.B. Lignin-Suylfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im falle der oralen Anwendung können Tabletten selbstverständlich außer den genannten Trägerstoffen auch Zusätze wie Natriumcitrat, Calciumcarbonat, und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren Mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erweisen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 m/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Die erfindungsgemäßen substituierten Benzylether können sowohl in der Humanmedizin als auch in der Veterinärmedizin angewendet werden.

Herstellungsbeispiele

Die Retentionszeiten $R_f$ (min) werden mit einem HPLC-Gerät (Fa. Knauer) an Hiber-Säulen (Fa. Merck) ermittelt.

System a: RP—8,5 µm,
Durchfluß: 1,5 ml/min,
Eluens: Acetonitril/Wasser=70:30 (v/v)

System b: RP—8,7 µm,
Durchfluß: 2,0 ml/min,
Eluens: Acetonitril/Wasser=70:30 (v/v)

Beispiel 1

1-(3-Methylphenyl)pentanol

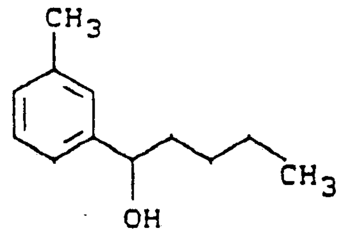

Unter Stickstoff werden 13.4 g Magnesium in 200 ml absolutem Ether vorgelegt. 75.4 g n-Butylbromid, in 300 ml absolutem Ether gelöst, werden so zugetropft, daß die Reaktionslösung siedet.

Danach wird 1 h im Rückfluß erhitzt und anschließend auf 0°C abgekühlt. 60 g 3-Methylbenzaldehyd gelöst in 250 ml absolutem Ether werden bei 0°C zugetropft. Man läßt die Reaktionslösung über Nacht stehen und gießt dann auf 1 l Eiswasser. Das Reaktionsgemisch wird mit 2 n Salzsäure angesäuert. Die organische Phase wird abgetrennt, die wässrige Phase 2 mal mit je 300 ml Essigester extrahiert und die vereinigten organischen Phasen werden 2 mal mit je 200 ml Wasser gewaschen, über $MgSO_4$ getrocket und in Vakuum einrotiert. Der Rückstand wird bei 64—66°C/0,02 mm destilliert.

Ausbeute: 93% der Theorie.

Analog Beispiel 1 wurden folgende Beispiele hergestellt:

Beispiel 2

1-(2-Methylphenyl)pentanol

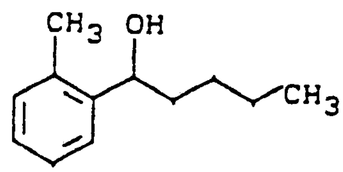

Ausbeute: 84,5% der Theorie
Siedepunkt: 110°C/0.1 mm (Kugelrohr)

Beispiel 3

1-(2-Methylphenyl)heptanol

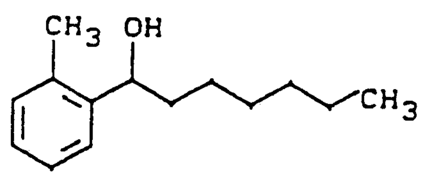

Ausbeute: 95% der Theorie
Siedepunkt: 115°C/0.1 mm (Kugelrohr)

Beispiel 4

1-(3-Methylphenyl)heptanol

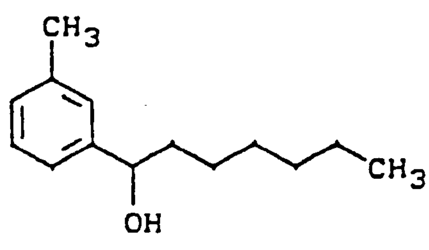

Ausbeute: 91% der Theorie
Siedepunkt: 80—82°C/0.1 mm

Beispiel 5

1-(2-Methylphenyl)hexanol

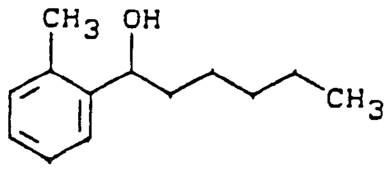

Ausbeute: 94% der Theorie
Siedepunkt: 88°C/0.06 mm

Beispiel 6

1-(3-Methylphenyl)hexanol

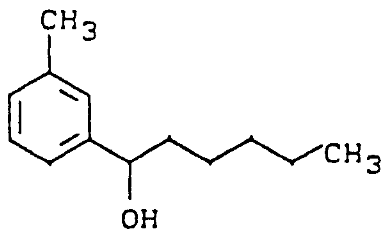

Ausbeute: 88% der Theorie
Siedepunkt: 85—90°C/0.06 mm

Beispiel 7

1-(4-Methylphenyl)pentanol

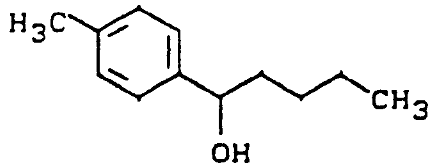

Ausbeute: 71% der Theorie
Siedepunkt: 60—64°C/0.08 mm

Beispiel 8

3,3-Dimethyl-1-(3-methylphenoxy)-2-butanol

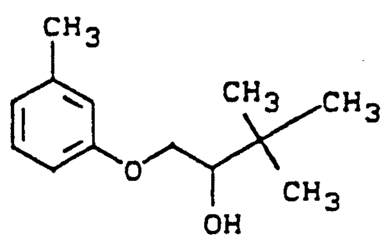

108 g 3-Hydroxytoluol und 101 g Triethylamin werden auf 120°C erwärmt. Bei dieser Temperatur werden innerhalb von 2.5 h 100.2 g 3,3-Dimethyl-1,2-butenoxid zugetropft und 15 h bei 120°C gerührt. Nach Abkühlen werden 500 ml Dichlormethan zugegeben und man wäscht 3 mal mit jeweils 500 ml 2 n HCl, 1 mal mit 200 ml gesättigter Natriumhydrogencarbonat-Lösung, 2 mal mit 200 ml Wasser. Die organische Phase wird über $Na_2SO_4$ getrocknet, im Vakuum eingeengt und der Rückstand bei 96°C/0.15 mm destilliert.

Ausbeute: 76% der Theorie

Analog Beispiel 8 wurden hergestellt:

Beispiel 9

1-(2-Methylphenoxy)-2-butanol

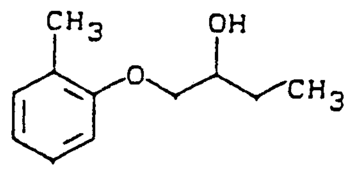

Ausbeute: 93% der Theorie
Siedepunkt: 69—73°C/0.03 mm

Beispiel 10

1-(3-Methylphenoxy)-2-butanol

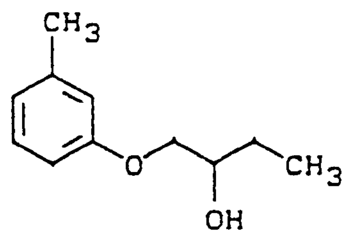

Ausbeute: 91% der Theorie
Siedepunkt: 69—75°C/0.015 mm

Beispiel 11

2-(3-Methylphenoxy)cyclohexanol (trans-Form)

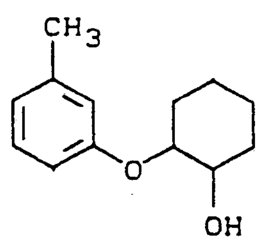

Ausbeute: 47% der Theorie
Siedepunkt: 130—132°C/0.5 mm

Beispiel 12

1-(3-methylphenoxy)-2-hexanol

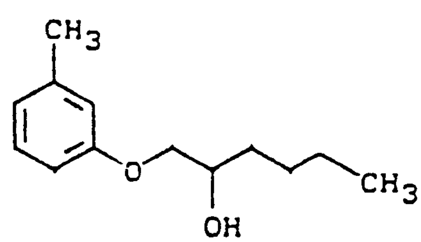

Ausbeute: 59% der Theorie
Siedepunkt: 120—124°C/0.9 mm

Beispiel 13

Essigsäure-1-(3-methylphenyl)pentylester

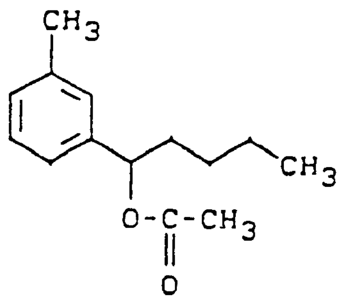

65.4 g 3-(1-Hydroxypentyl)toluol werden bei 0°C in 500 ml absolutem Dichlormethan gelöst und nacheinander mit 104 ml Acetanhydrid, 89 ml Pyridin und 1 g Dimethylaminopyridin versetzt. Die Reaktionslösung wird 1 h bei 0°C, 2 h bei 25°C gerührt und dann auf 1 l Eiswasser gegossen. Nach Abtrennen der organischen Phase wird 3 mal mit je 200 ml Dichlormethan extrahiert. Die organischen Phasen werden 3 mal mit je 200 ml 2 n HCl, 3 mal mit je 200 ml gesättigter $NaHCO_3$, 2 mal mit je 200 ml Wasser gewaschen, über Magnesiumsulfat getrocknet und im Vakuum eingeengt. Der Rückstand wird bei 95°C/0.1 mm (Kugelrohr) destilliert.

Ausbeute: 95% der Theorie
Siedepunkt: 95°C/0.1 mm (Kugelrohr)

Analog Beispiel 13 wurden hergestellt:

Beispiel 14

Essigsäure-1-(2-methylphenyl)pentylester

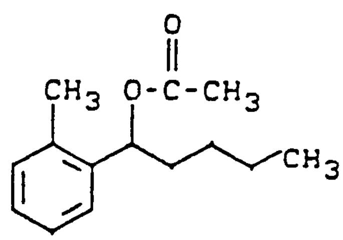

Ausbeute: 93% der Theorie
Siedepunkt: 90°C/0.1 mm (Kugelrohr)

Beispiel 15

Essigsäure-1-(2-methylphenyl)heptylester

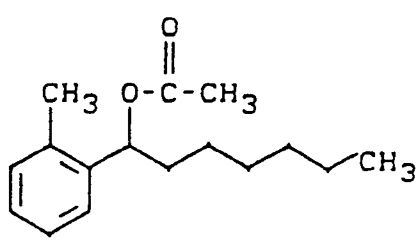

Ausbeute: 94% der Theorie
Siedepunkt: 80—83°C/0.1 mm

Beispiel 16

Essigsäure-1-(3-methylphenyl)heptylester

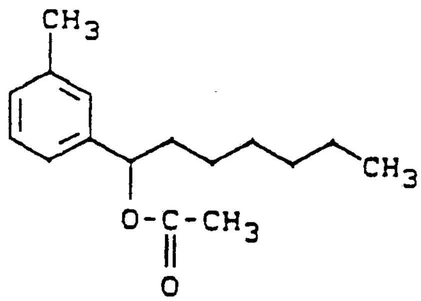

Ausbeute: 96% der Theorie
Siedepunkt: 83—86°C/0.07 mm

Beispiel 17

Essigsäure-1-(2-methylphenyl)hexylester

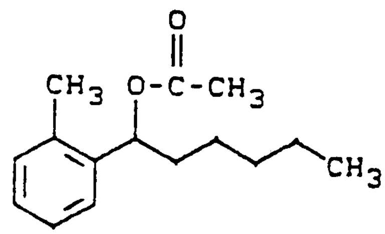

Ausbeute: 87% der Theorie
Siedepunkt: 73—78°C/0.005 mm

Beispiel 18

Essigsäure-1-(3-methylphenyl)hexylester

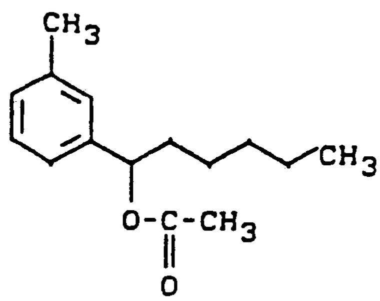

Ausbeute: 86% der Theorie
Siedepunkt: 75—78°C/0.005 mm

Beispiel 19

Essigsäure-1-[(2-methylphenoxy)methyl]propylester

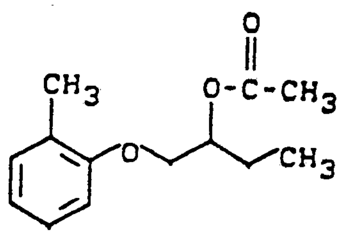

Ausbeute: 85% der Theorie
Siedepunkt: 72—74°C/0.015 mm

Beispiel 20

Essigsäure-1-[(3-methylphenoxy)methyl]propylester

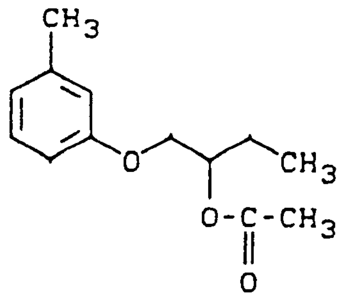

Ausbeute: 90% der Theorie
Siedepunkt: 75—80°C/0.015 mm

Beispiel 21

Essigsäure-2-(3-methylphenoxy)cyclohexylester (trans-Form)

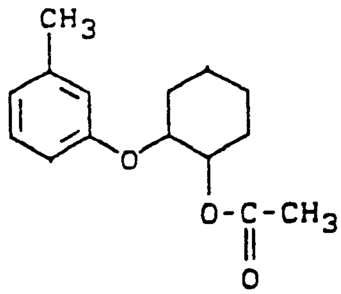

Ausbeute: 86% der Theorie
Siedepunkt: 102—106°C/0.2 mm

Beispiel 22

Essigsäure-2,2-dimethyl-1-[(3-methylphenoxy)methyl]propylester

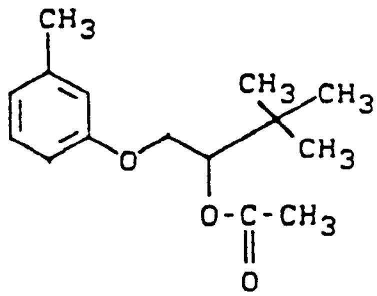

Ausbeute: 91% der Theorie
Siedepunkt: 102°C/0.2 mm

Beispiel 23

Essigsäure-1-[(3-methylphenoxy)methyl]pentylester

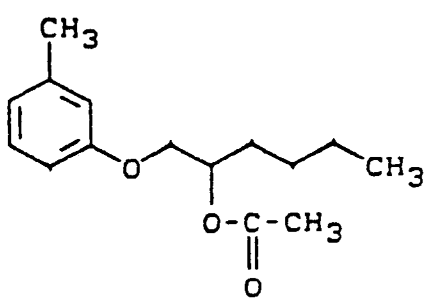

Ausbeute: 89% der Theorie
Siedepunkt: 106—107°C/0.2 mm

Beispiel 24

Essigsäure-1-(4-methylphenyl)pentylester

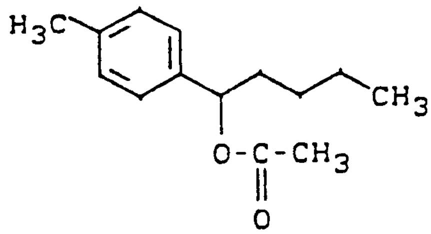

Ausbeute: 83% der Theorie
Siedepunkt: 61—64°C/0.03 mm

Beispiel 25

Essigsäure-1-(3-brommethylphenyl)pentylester

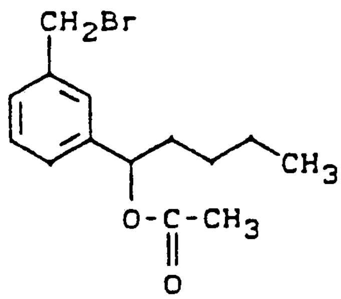

11 g 3-(1-Acetoxy-pentyl)toluol werden in 100 ml absolutem Tetrachlorkohlenstoff gelöst. Nach Zugabe von 8,9 g N-Bromsuccinimid und 200 mg Azobisisobutyronitril wird langsam bis zum Rückfluß erwärmt. Nach Beendigung der Reaktion wird noch 1 h im Rückfluß erhitzt, danach auf 0°C abgekühlt und vom Succinimid abfiltriert. Nach Einengen im Vakuum wird der Rückstand bei 97—102°C/0.08 mm destilliert.

Ausbeute: 73% der Theorie

Analog Beispiel 25 wurden hergestellt:

Beispiel 26

Essigsäure-1-(2-brommethylphenyl)pentylester

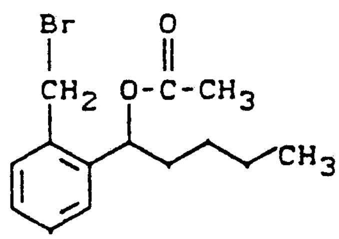

Ausbeute: 72% der Theorie
Siedepunkt: 165°C/0.1 mm (Kugelrohr)

Beispiel 27

Essigsäure-1-(2-brommethylphenyl)heptylester

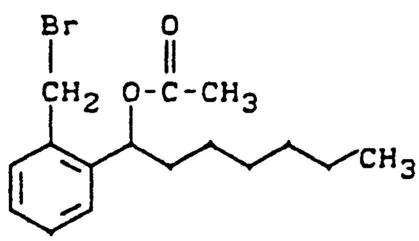

Ausbeute: 83% der Theorie
Siedepunkt: 120°C/0.05 mm

Beispiel 28

Essigsäure-1-(3-brommethylphenyl)heptylester

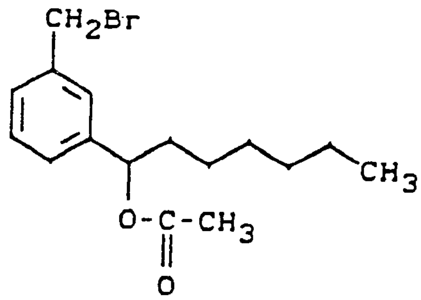

Ausbeute: 75% der Theorie
Siedepunkt: 122°C/0.05 mm

Beispiel 29

Essigsäure-1-(2-brommethylphenyl)heptylester

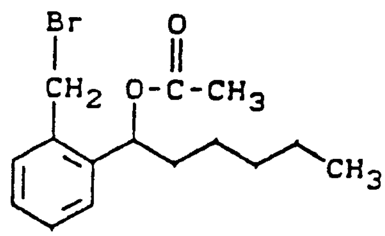

Ausbeute: 75% der Theorie
Siedepunkt: 105°C/0.01 mm

Beispiel 30

Essigsäure-1-(3-brommethylphenyl)hexylester

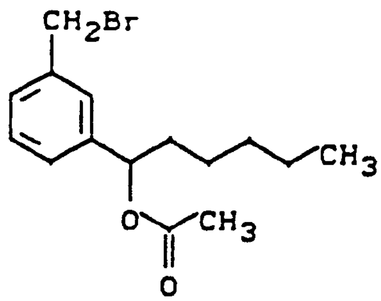

Ausbeute: 84% der Theorie
Siedepunkt: 128°C/0.03 mm

Beispiel 31

Essigsäure-1-[(2-brommethylphenoxy)methyl]propylester

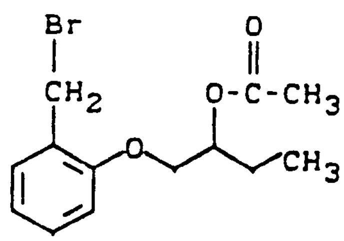

Ausbeute: 82% der Theorie
Siedepunkt: 119—123°C/0.03 mm

Beispiel 32

Essigsäure-1-[(3-brommethylphenoxy)methyl]propylester

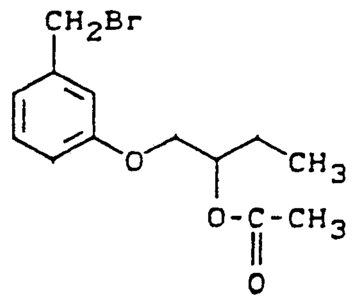

Ausbeute: 70% der Theorie
Siedepunkt: 85—89°C/0.05 mm

Beispiel 33

Essigsäure-2-(3-brommethylphenoxy)cyclohexylester (trans-Form)

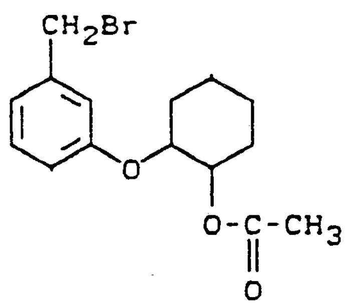

Ausbeute: 65% der Theorie
Siedepunkt: 150—157°C/0.2 mm

Beispiel 34

Essigsäure-1-[(3-brommethylphenoxy)methyl]-2,2-dimethylpropylester

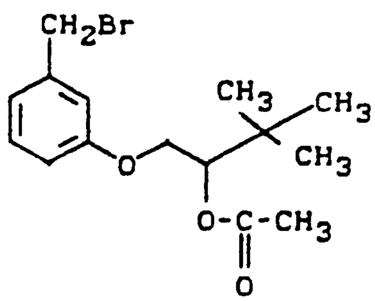

Ausbeute: 58% der Theorie
Siedepunkt: 158—160°C/0.2 mm

Beispiel 35

Essigsäure-1-[(3-brommethylphenoxy)methyl]pentylester

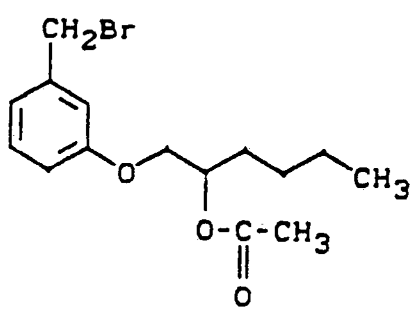

Ausbeute: 57% der Theorie
Siedepunkt: 146—156°C/0.2 mm

Beispiel 36

Essigsäure-1-(4-brommethylphenyl)pentylester

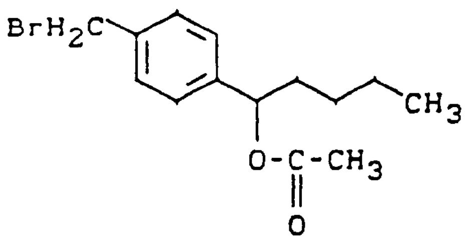

Ausbeute: 46% der Theorie
Siedepunkt: 112°C/0.005 mm

Beispiel 37

Essigsäure-1-[3-(chinolin-8-yloxymethyl)phenyl]pentylester

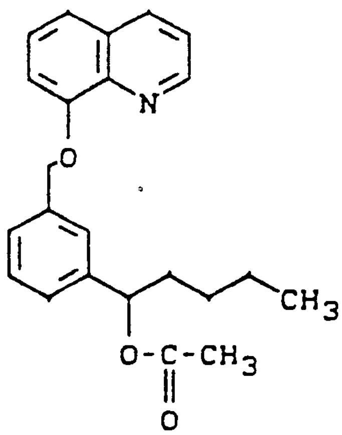

1,45 g 8-Hydroxychinolin und 1.38 g gemahlenes, wasserfreies Kaliumcarbonat werden in 20 ml Dimethylformamid 2 h bei 25°C gerührt. Dann werden 2.99 g Essigsäure-1-(3-brommethylphenyl)pentylester, gelöst in 10 ml Dimethylformamid, zugetropft und die Reaktionsmischung über Nacht gerührt. Das Lösungsmittel wird im Vakuum abdestilliert, der Rückstand zwischen Wasser/Essigester verteilt, die organische Phase über $Na_2SO_4$ getrocknet und anschleißend im Vakuum eingeengt. Der Rückstand wird über Kieselgel 60 (Merck 9385) mit $CH_2Cl_2$/Methanol 100:5 chromatographiert ($R_f$ = 0.45). Man erhält ein Öl in einer Ausbeute von 94% der Theorie.

$R_f$ = 3.17 (System a)

Analog Beispiel 37 wurden hergestellt:

Beispiel 38

Essigsäure-1-[3-(chinolin-8-yloxymethyl)phenyl]hexylester

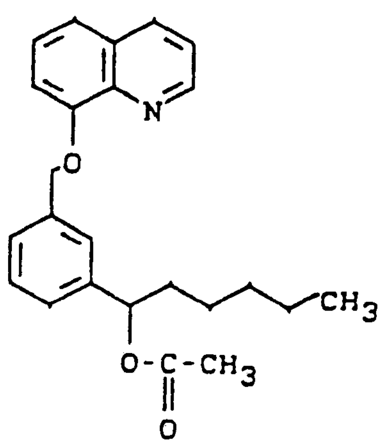

Ausbeute: 68% der Theorie
$R_f$ = 3.28 (System b)

Beispiel 39

Essigsäure-1-[3-(chinolin-8-yloxymethyl)phenyl]heptylester

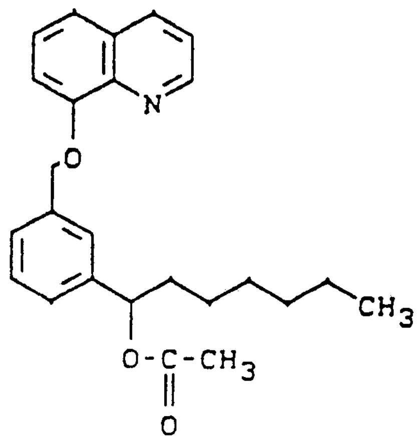

Ausbeute: 62% der Theorie
$R_f$ = 3.89 (System b)

Beispiel 40

Essigsäure-1-[3-(5,7-dichlorchinolin-8-yloxymethyl)phenyl]heptylester

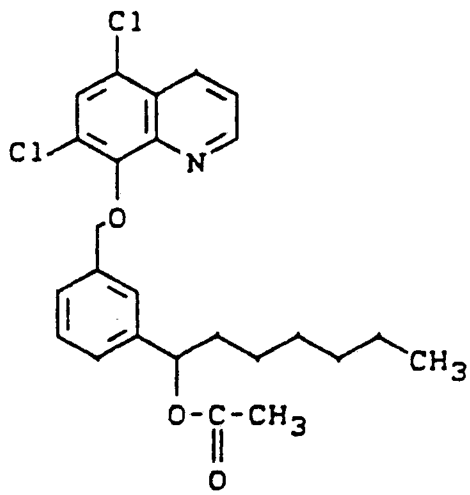

Ausbeute: 69% der Theorie
$R_f$ = 7.88 (System a)

Beispiel 41

Essigsäure-1-[2-(chinolin-8-yloxymethyl)phenyl]pentylester

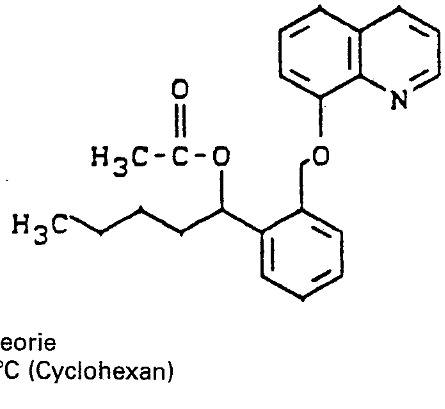

Ausbeute: 83% der Theorie
Schmelzpunkt: 80—82°C (Cyclohexan)
$R_f$ = 3.29 (System a)

Beispiel 42

Essigsäure-1-[2-(chinolin-8-yloxymethyl)phenyl]hexylester

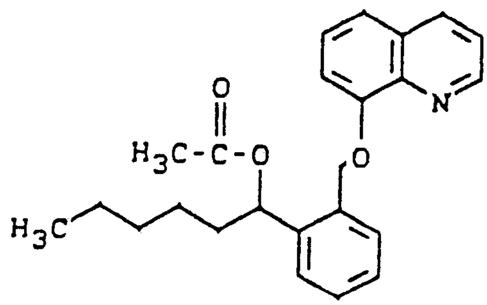

Ausbeute: 66% der Theorie
Schmelzpunkt: 79—81°C (Cyclohexan)
$R_f$ = 3.86 (System a)

Beispiel 43

Essigsäure-1-[2-(5,7-dichlorchinolin-8-yloxymethyl)phenyl]pentylester

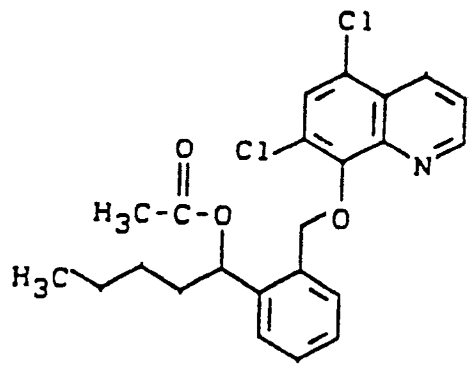

Ausbeute: 77% der Theorie
$R_f$ = 6.19 (System a)

Beispiel 44

Essigsäure-1-[3-(chinaldin-8-yloxymethyl)phenyl]hexylester

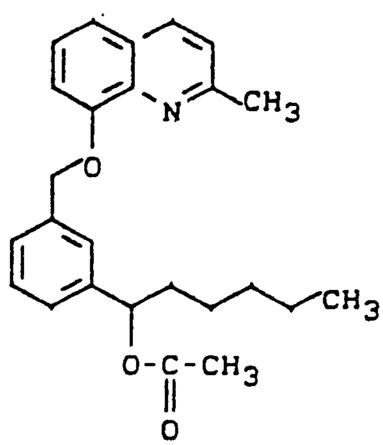

Ausbeute: 67% der Theorie
$R_f$ = 3.71 (System b)

Beispiel 45

Essigsäure-1-[3-(5,7-dichlorchinolin-8-yloxymethyl)phenyl]pentylester

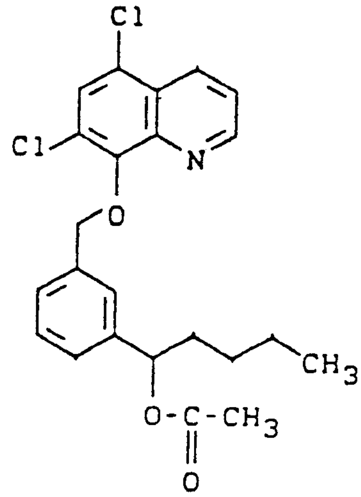

Ausbeute: 50% der Theorie
$R_f$ = 5.52 (System a)

Beispiel 46

Essigsäure-2-[3-(chinolin-8-yloxymethyl)phenoxy]cyclohexylester (trans-Form)

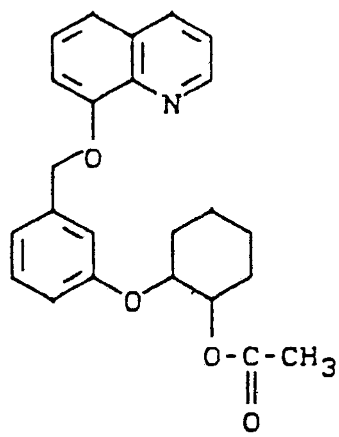

Ausbeute: 77% der Theorie
$R_f$ = 2.47 (System b)

Beispiel 47

Essigsäure-1-[3-(chinolin-8-ylaminomethyl)phenyl]pentylester

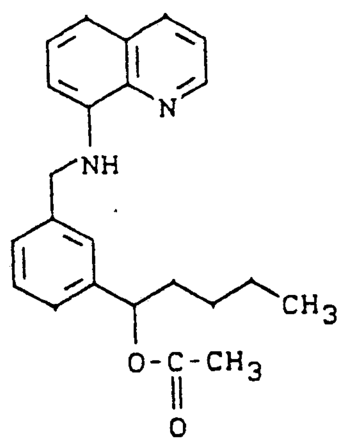

Ausbeute: 63% der Theorie

Beispiel 48

Essigsäure-1-[3-(isochinolin-5-yloxymethyl)phenyl]pentylester

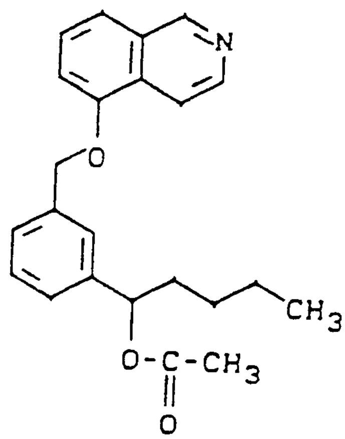

Ausbeute: 46% der Theorie
$R_f$ = 4.08 (System b)

Beispiel 49

Essigsäure-1-[4-(chinolin-8-yloxymethyl)phenyl]pentylester

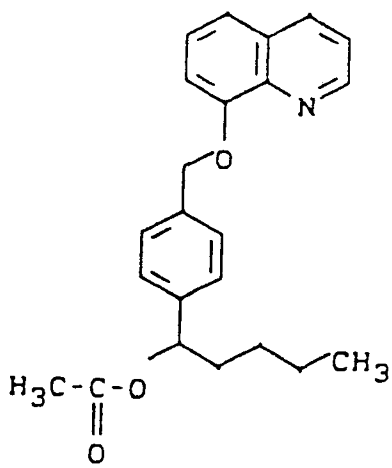

Ausbeute: 75% der Theorie
$R_f$ = 3.06 (System b)

Beispiel 50

Essigsäure-1-[3-(chinolin-8-ylthiomethyl)phenyl]pentylester

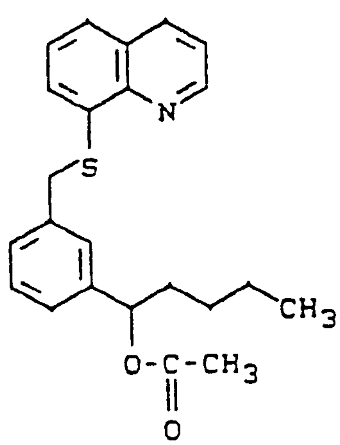

Ausbeute: 95% der Theorie
$R_f$ = 3.26 (System b)

Beispiel 51

Essigsäure-1-[3-(chinolin-8-yloxymethyl)phenoxymethyl]-2,2-dimethyl-propylester

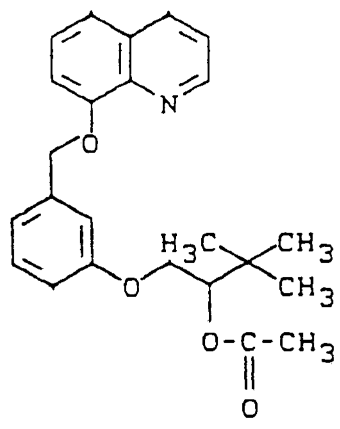

Ausbeute: 93% der Theorie
$R_f$ = 2.74 (System b)

Beispiel 52

Essigsäure-1-[3-(chinolin-8-yloxymethyl)phenoxymethyl]pentylester

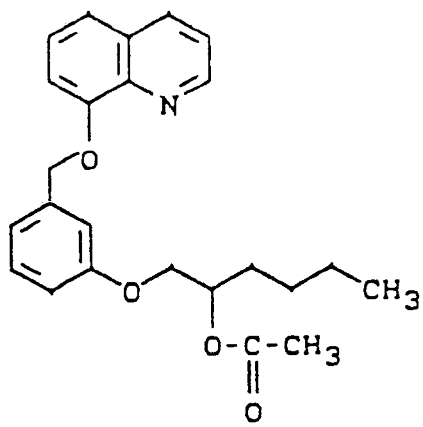

Ausbeute: 70% der Theorie
Schmelzpunkt: 85—86°C (Essigester/Cyclohexan)
$R_f$ = 2.97 (System b)

Beispiel 53

Essigsäure-1-[3-(chinolin-8-yloxymethyl)phenoxymethyl]propylester

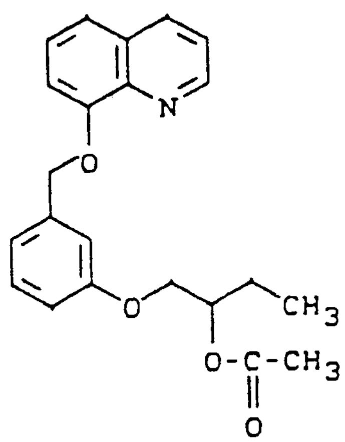

Ausbeute: 61% der Theorie
Schmelzpunkt: 77—79°C (Essigester/Cyclohexan)
$R_f$ = 2.33 (System b)

Beispiel 54

Essigsäure-1-[2-(chinolin-8-yloxymethyl)phenoxymethyl]propylester

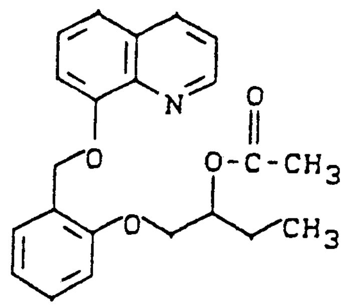

Ausbeute: 84% der Theorie

$R_f$ = 2.38 (System b)

Beispiel 55

1-[3-(Chinolin-8-yloxymethyl)phenyl]pentanol

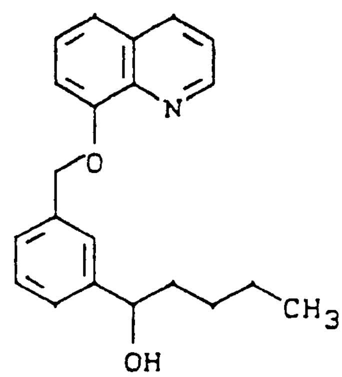

3.63 g Essigsäure-1-[3-(8-chinoyloxymethyl)phenyl]pentylester werden in 50 ml Methanol gelöst. Nach Zugabe von 10 ml 2 n NaOH wird 15 h bei 25°C gerührt, das organische Lösungsmittel in Vakuum abrotiert und die wässrige Phase 3 mal mit je 20 ml Ether extrahiert. Die organischen Phasen werden 2 mal mit je 30 ml Wasser gewaschen, über $Na_2SO_4$ getrocknet und im Vakuum eingeengt. Der Rückstand wird über Kieselgel 60 (Merch 9385) chromatographiert

$R_f$ = 2.53 (System a)

Ausbeute: 91% der Theorie

Schmelzpunkt: 72 bis 73°C (Diisopropylether)

Analog Beispiel 55 wurden hergestellt:

Beispiel 56

1-[3-(Chinolin-8-yloxymethyl)phenyl]hexanol

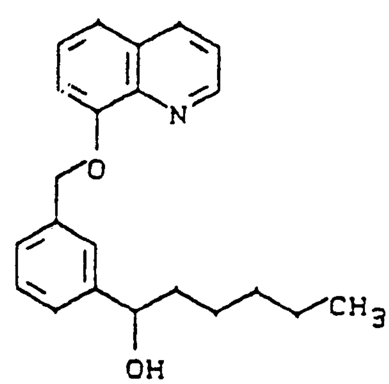

Ausbeute: 78% der Theorie

$R_f$ = 2.33 (System b)

Beispiel 57

1-[3-(Chinolin-8-yloxymethyl)phenyl]heptanol

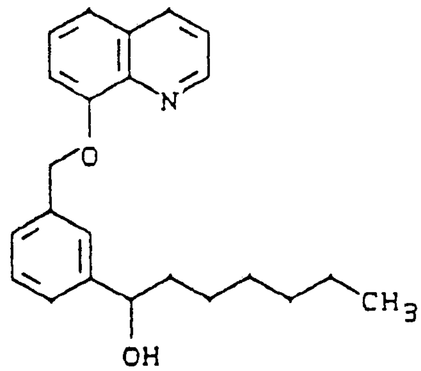

Ausbeute: 77% der Theorie
$R_f$ = 2.75 (System b)

Beispiel 58

1-[3-(5,7-Dichlorchinolin-8-yloxymethyl)phenyl]heptanol

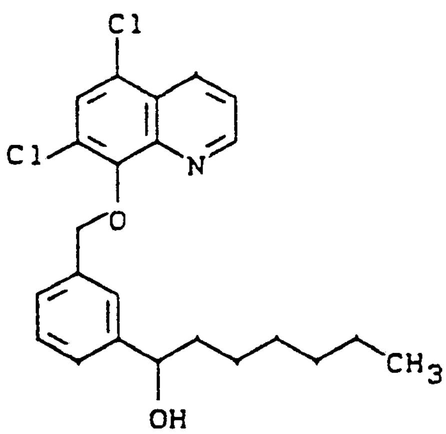

Ausbeute: 86%
$R_f$ = 5.16 (System a)

Beispiel 59

1-[2-(Chinolin-8-yloxymethyl)phenyl]pentanol

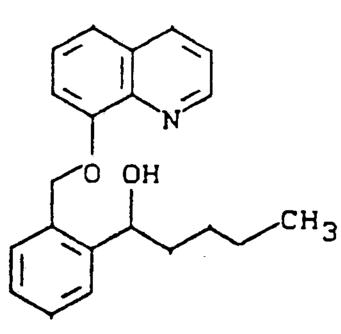

Ausbeute: 80% der Theorie
$R_f$ = 2.90 (System a)

Beispiel 60

1-[2-(Chinolin-8-yloxymethyl)phenyl]hexanol

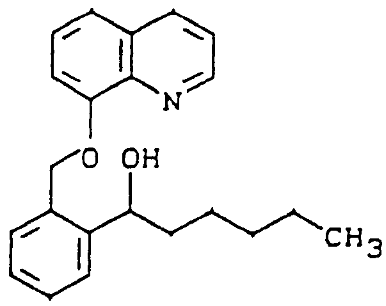

Ausbeute: 81% der Theorie
$R_f$ = 2.71 (System b)

Beispiel 61

1-[3-(2-Methylchinolin-8-yloxymethyl)phenyl]hexanol

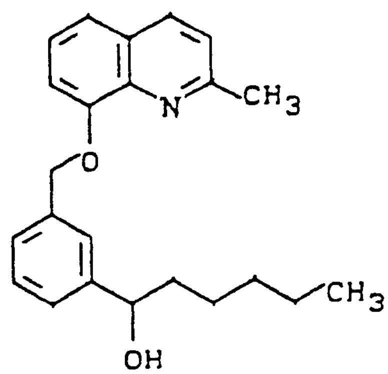

Ausbeute: 86% der Theorie
$R_f$ = 2.54 (System b)

Beispiel 62

1-[3-(Isochinolin-5-yloxymethyl)phenyl]pentanol

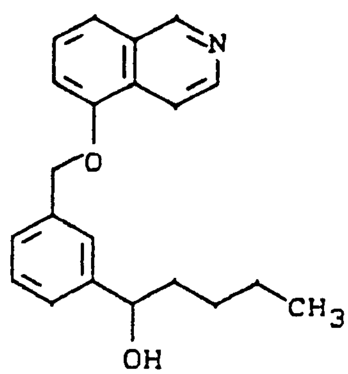

Ausbeute: 16% der Theorie
$R_f$ = 0.3 ($CH_3Cl_2$:$CH_3OH$ = 100:2)

Beispiel 63

1-[3-(Chinolin-8-ylthiomethyl)phenyl]pentanol

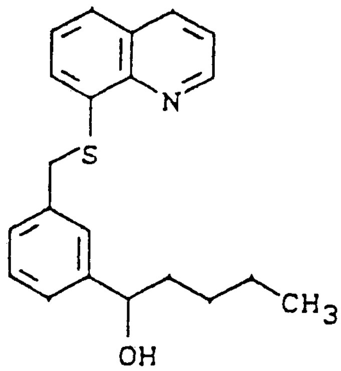

Ausbeute: 63% der Theorie
$R_f$ = 2.29 (System b)

Beispiel 64

1-[3-(Chinolin-8-ylaminomethyl)phenyl]pentanol

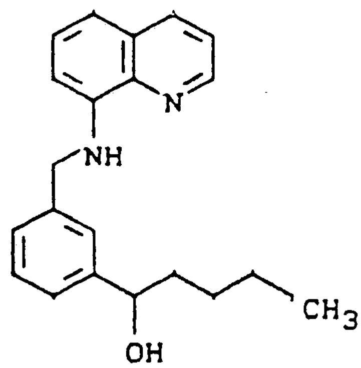

Ausbeute: 51% der Theorie

Beispiel 65

2-[3-(Chinolin-8-ylthiomethyl)phenoxy]cyclohexanol (trans-Form)

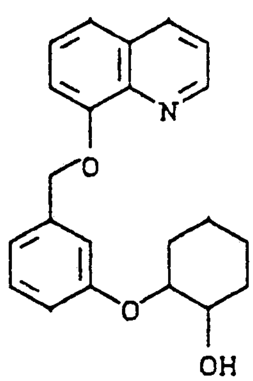

Ausbeute: 65% der Theorie
Schmelzpunkt: 109—110°C (Essigester/Cyclohexan)
$R_f$ = 1.90 (System b)

Beispiel 66

1-[2-(Chinolin-8-yloxymethyl)phenoxymethyl]propanol

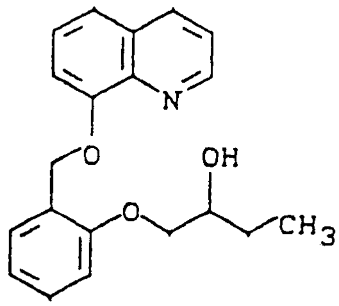

Ausbeute: 83% der Theorie
$R_f$ = 1.87 (System b)

Beispiel 67

1-[3-(Chinolin-8-yloxymethyl)phenoxymethyl]propanol

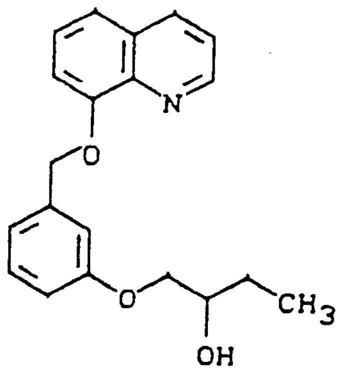

Ausbeute: 98% der Theorie
$R_f$ = 1.75 (System b)

Beispiel 68

1-[3-(Chinolin-8-yloxymethyl)phenoxymethyl]pentanol

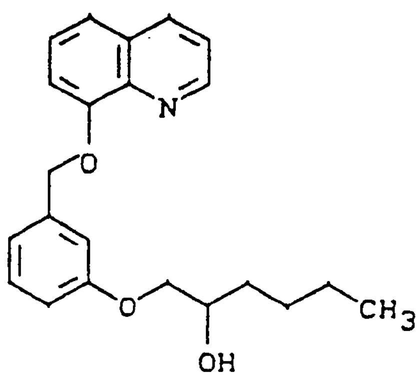

Ausbeute: 98% der Theorie
$R_f$ = 2.10 (System b)

Beispiel 69

1-[3-(Chinolin-8-yloxymethyl)phenoxymethyl]-2,2-dimethylpropanol

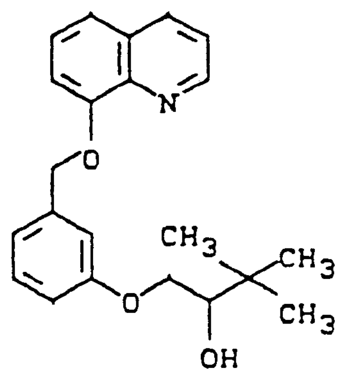

Ausbeute: 95%
Schmelzpunkt: 103—105°C
$R_f$ = 2.07 (System b)

Beispiel 70

Essigsäure-1-[2-(chinolin-N-oxid-8-yloxymethyl)phenyl]hexylester

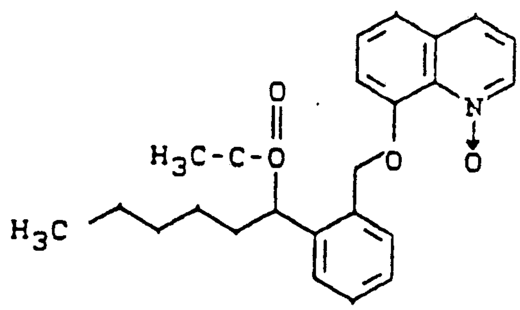

1.4 g Essigsäure-1-[2-(chinolin-8-yloxymethyl)phenyl]hexylester werden in 20 ml Chloroform gelöst. Bei 0°C werden 0.64 g m-Chlorperpenzoesäure (80%ig) in 10 ml Chloroform gelöst, in 30 min. zugetropft. Es wird 15 h bei 25°C gerührt, das Lösungsmittel abgedampft und der Rückstand über Kieselgel 60 (Merck 7734) mit Essigester chromatographiert. Man erhält ein Öl.

Ausbeute: 69% der Theorie
$R_f$ = 2.89 (System b)

Beispiel 71

1-[2-(Chinolin-N-oxid-8-yloxymethyl)phenyl]hexanol

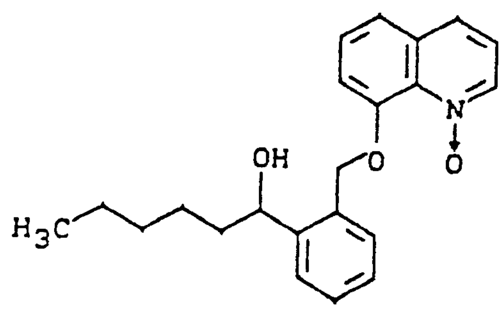

2.2 g 1-[2-(Chinolin-8-yloxymethyl)phenyl]hexanol werden in 20 ml Chloroform gelöst. Bei 0°C werden 0.64 g m-Chlorperbenzoesäure, in 15 ml Chloroform gelöst, in 30 min zugetropft. Es wird 15 h bei 25°C gerührt, das Lösungsmittel abgedampft und der Rückstand über Kieselgel 60 (Merck 7734) mit Methylenchlorid/Methanol = 100:5 chromatographiert. Das Produkt kristallisiert aus.

Ausbeute: 26% der Theorie.
Schmelzpunkt: 99—101°C
$R_f$ = 2.32 (System b)

Beispiel 72

Essigsäure-1-[2-(2-(2(1H)-chinolon-8-yloxymethyl)phenyl]heptylester

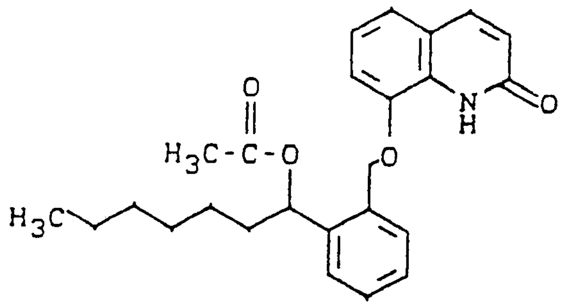

6.5 g 2,8-Dihydroxychinolin und 5.5 g wasserfreies, gemahlenes Kaliumcarbonat werden in 100 ml Dimethylformamid 1 h bei 25°C gerührt. Nach Zugabe von 13.1 g Essigsäure-1-(2-brommethylphenyl)heptylester in 50 ml Dimethylformamid wird bei 25°C 20 h gerührt. Das Lösungsmittel wird im Vakuum entfernt, der Rückstand in 100 ml Wasser aufgenommen und 3 mal mit je 50 ml Essigester extrahiert. Die organischen Phasen werden 1 mal mit je 50 ml Wasser gewaschen, über $Na_2SO_4$ getrocknet und im Vakuum eingeengt. Das verbleibende Öl wird über Kieselgel 60 mit $CH_2Cl_2$/Essigester 10:1 chromatographiert. Das erhaltene Produkt kristallisiert langsam aus.

Ausbeute: 74% der Theorie
Schmelzpunkt: 113—115°C
$R_f$ = 3.32 (System b)

Analog Beispiel 72 werden hergestellt:

Beispiel 73

Essigsäure-1-[2-(2-(2(1H)-chinolon-8-yloxymethyl)phenyl]hexylester

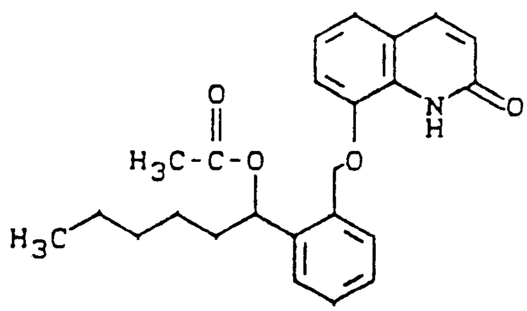

Ausbeute: 43% der Theorie
Schmelzpunkt: 92—95°C (Cyclohexan)
$R_f$ = 2.86 (System b)

Beispiel 74

Essigsäure-1-[3-(2(1H)-chinolon-8-yloxymethyl)phenyl]heptylester

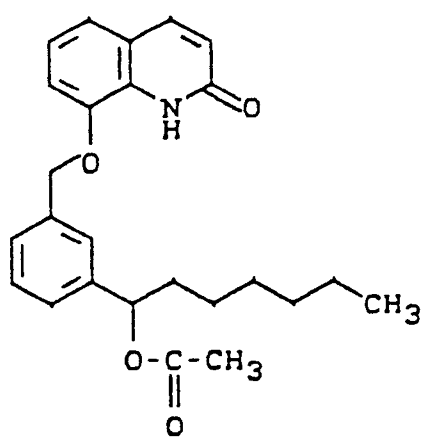

Ausbeute: 50% der Theorie
$R_f$ = 3.20 (System b)

Beispiel 75

Essigsäure-1-[3-(2(1H)-chinolon-8-yloxymethyl)phenyl]hexylester

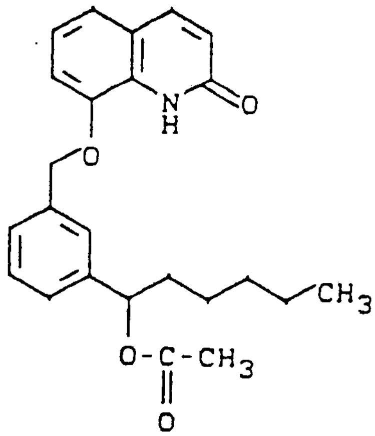

Ausbeute: 48% der Theorie
$R_f$ = 2.73 (System b)

Beispiel 76

Essigsäure-2-[3-(2-(2(1H)-chinolon-8-yloxymethyl)phenoxy]cyclohexylester (trans-Form)

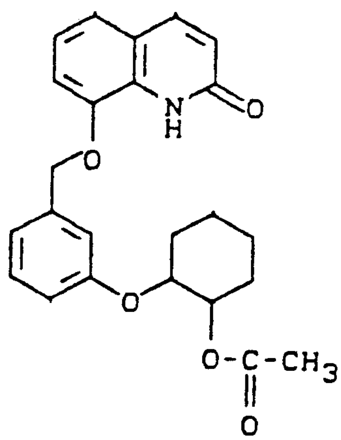

Ausbeute: 67% der Theorie
$R_f$ = 2.12 (System b)

Beispiel 77

Essigsäure-1-[2-(2(1H)-chinolon-8-yloxymethyl)phenoxymethyl]propylester

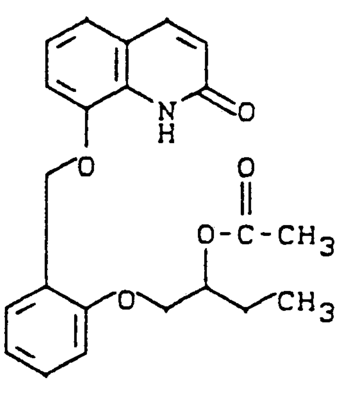

Ausbeute: 48% der Theorie
Schmelzpunkt: 112—114°C (Essigester/Cyclohexan)
$R_f$ = 2.07 (System b)

Beispiel 78

Essigsäure-1-[3-(2(1H)-Chinolon-8-yloxymethyl)phenoxymethyl]propylester

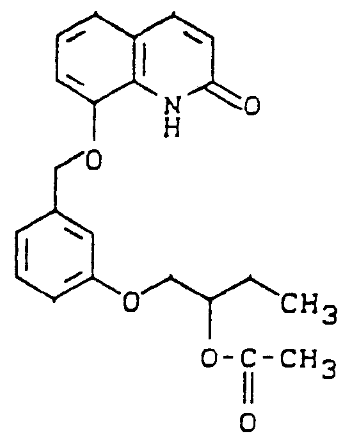

Ausbeute: 65% der Theorie
Schmelzpunkt: 81—84°C (Essigester/Cyclohexan)
$R_f$ = 2.01 (System b)

Beispiel 79

1-[2-(2(1H)-Chinolon-8-yloxymethyl)phenyl]heptanol

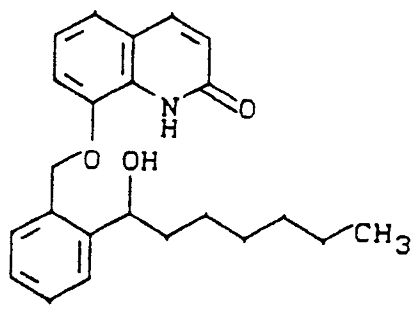

6.0 g Essigsäure-1-[2-(2(1H)-chinolin-8-yloxymethyl)phenyl]-heptylester werden in 150 ml Methanol gelöst und mit 60 ml 1 n NaOH versetzt. Es wird 15 h refluxiert. Nach Abkühlen werden 60 ml 1n HCl zugegeben, vom ausgefallenen Produkt abgesaugt und aus Isopropanol umkristallisiert.
Ausbeute: 50% der Theorie
Schmelzpunkt: 195—197°C (Isopropanol)
$R_f$ = 2.33 (System b)
Analog Beispiel 58 wurden hergestellt:

Beispiel 80

1-[2-(2(1H)-Chinolon-8-yloxymethyl)phenyl]hexanol

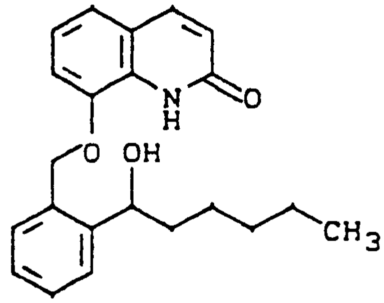

Ausbeute: 74% der Theorie
Schmelzpunkt: 201—202°C (Isopropanol)
$R_f$ = 2.06 (System b)

Beispiel 81

1-[3-(2(1H)-Chinolon-8-yloxymethyl)phenyl]heptanol

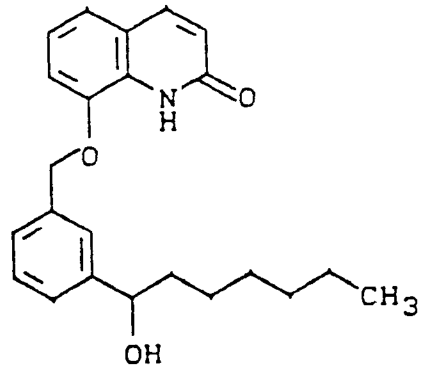

Ausbeute: 85% der Theorie
$R_f$ = 2.24 (System b)

Beispiel 82

1-[3-(2(1H)-Chinolon-8-yloxymethyl)phenyl]hexanol

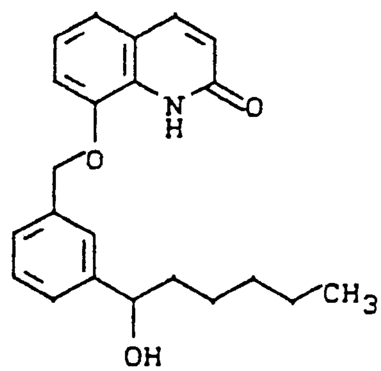

Ausbeute: 90% der Theorie
Schmelzpunkt: 97—99°C
$R_f$ = 1.94 (System b)

Beispiel 83

1-[3-(2(1H)-Chinolon-8-yloxymethyl)phenoxymethyl]propanol

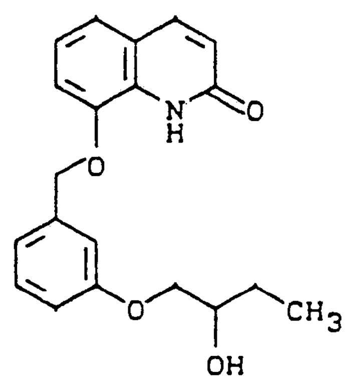

Ausbeute: 54% der Theorie
$R_f$ = 1.56 (System b)

Beispiel 84

2-[3-(2(1H)-Chinolon-8-yloxymethyl)phenoxy]cyclohexanol (trans-Form)

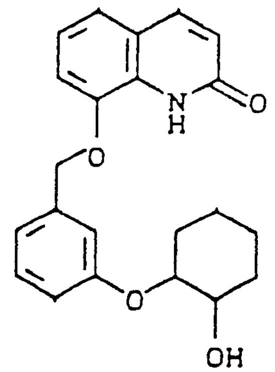

Ausbeute: 89% der Theorie
$R_f$ = 1.62 (System b)

Beispiel 85

1-[2-(2(1H)-Chinolon-8-yloxymethyl)phenoxymethyl]propanol

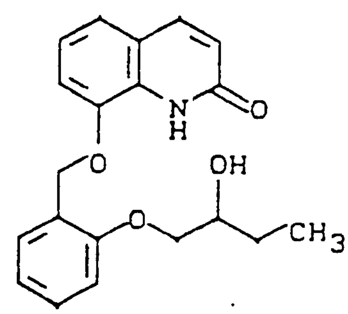

Ausbeute: 89% der Theorie
Schmelzpunkt: 114—116°C
$R_f$ = 1.66 (System b)

Analog Beispiel 1 wurden hergestellt:

Beispiel 86

3-Methyl-1-(3-Methylphenyl)butanol

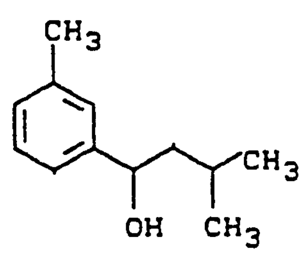

Ausbeute: 35% der Theorie
Siedepunkt: 72—76°C/0.008 mm
Analog Beispiel 23 wurden hergestellt:

Beispiel 87

Essigsäure-[3-methyl-1-(3-methylphenyl)]butylester

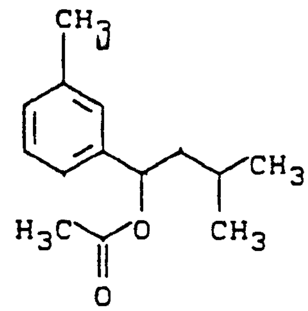

Ausbeute: 71% der Theorie
Siedepunkt: 54—56°C/0.005 mm

Beispiel 88

Popionsäure-1-(3-methylphenyl)]hexylester

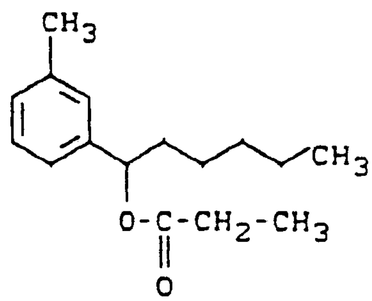

Ausbeute: 75% der Theorie
Siedepunkt: 78—80°C/0.005 mm
Analog Beispiel 25 wurden hergestellt:

Beispiel 89

Essigsäure-[1-(3-brommethylphenyl)-3-methyl]butylester

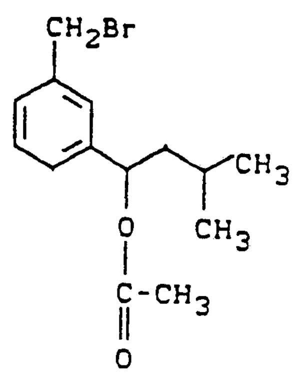

Ausbeute: 52%
Siedepunkt: 93°C/0.025 mm

Beispiel 90

Propionsäure-[1-(3-brommethylphenyl)]hexylester

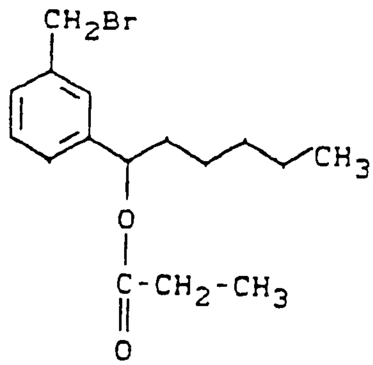

Ausbeute: 56% der Theorie
Siedepunkt: 111°C/0.02 mm
Analog Beispiel 37 wurden hergestellt:

Beispiel 91

Essigsäure-1-[3-(chinolin-8-yloxymethyl)phenyl]-3-methylbutylester

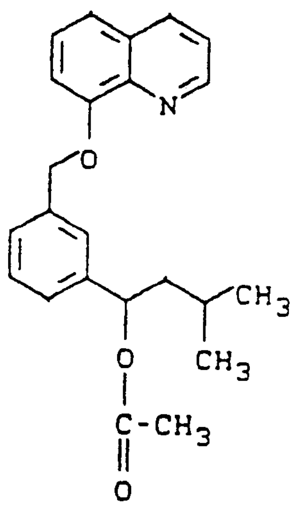

Ausbeute: 88% der Theorie
$R_f$ = 3.31 (System b)

Beispiel 92

Propionsäure-1-[3-(chinolin-8-yloxymethyl)phenyl]hexylester

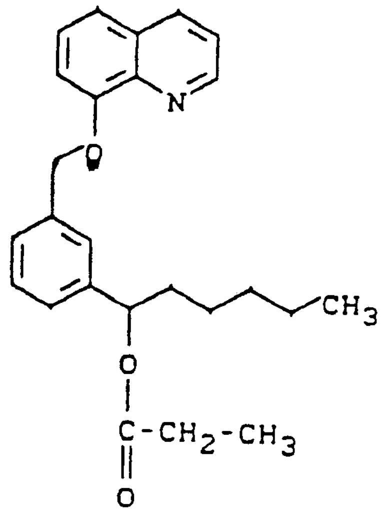

Ausbeute: 83% der Theorie
$R_f$ = 4.11 (System b)

Beispiel 93

Essigsäure-1-[3-(4-methylchinolin-8-yloxymethyl)phenyl]pentylester

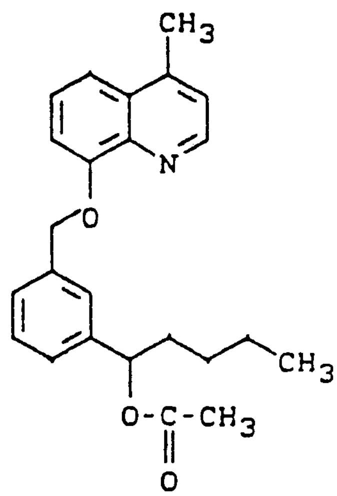

Ausbeute: 59% der Theorie
Schmelzpunkt: 67—70°C
$R_f$ = 2.95 (System b)

Beispiel 94

Essigsäure-1-[3-(4-chlorchinolin-8-yloxymethyl)phenyl]pentylester

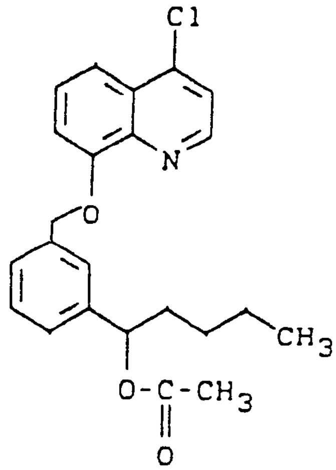

Ausbeute: 60% der Theorie
$R_f$ = 3.60 (System b)

Beispiel 95

Essigsäure-1-[3-(6-methylchinolin-8-yloxymethyl)phenyl]pentylester

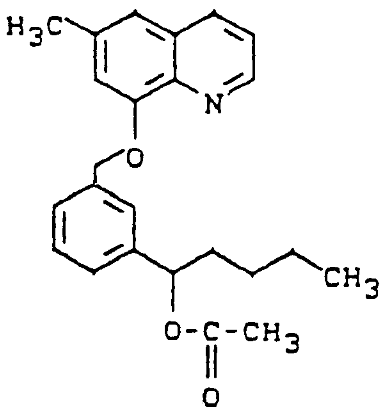

Ausbeute: 51% der Theorie
$R_f$ = 3.29 (System b)

Beispiel 96

Essigsäure-1-[3-(6-chlorchinolin-8-yloxymethyl)phenyl]pentylester

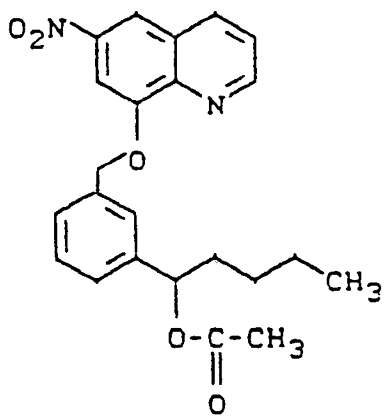

Ausbeute: 45% der Theorie
Schmelzpunkt: 72—74°C (Isopropanol)
$R_f$ = 2.74 (System b)

Beispiel 97

1-[4-(Chinolin-8-yloxymethyl)phenyl]pentanol

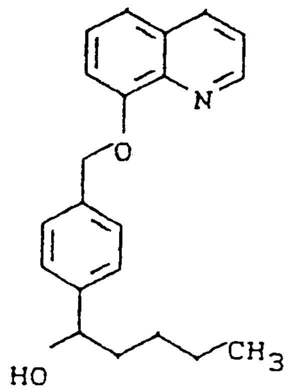

Ausbeute: 86% der Theorie
$R_f$ = 2.04 (System b)

Beispiel 98

1-[3-(Chinolin-8-yloxymethyl)phenyl]-3-methyl-butanol

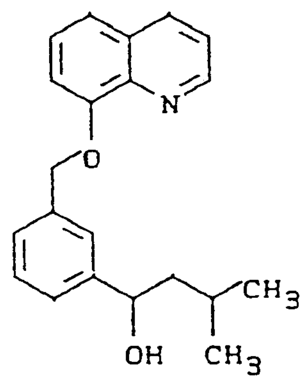

Ausbeute: 68% der Theorie
$R_f$ = 2.12 (System b)

Beispiel 99

1-[3-(4-Chlorchinolin-8-yloxymethyl)phenyl]pentanol

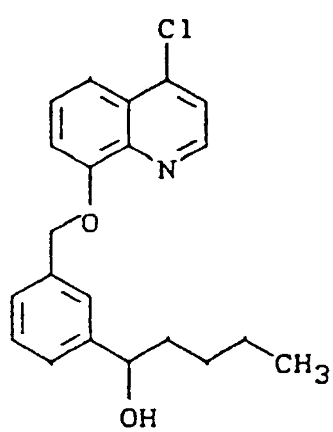

Ausbeute: 91% der Theorie
$R_f$ = 2.35 (System b)

Beispiel 100

1-[3-(4-Methylchinolin-8-yloxymethyl)phenyl]pentanol

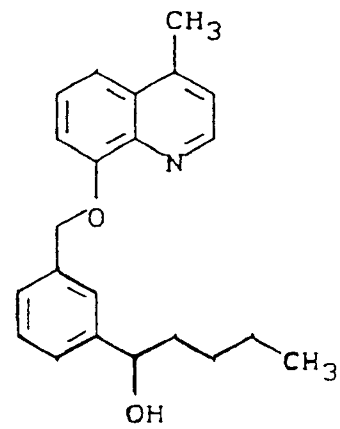

Ausbeute: 74% der Theorie
Schmelzpunkt: 94—96°C
$R_f$ = 2.21 (System b)

Beispiel 101

1-[3-(6-Methylchinolin-8-yloxymethyl)phenyl]pentanol

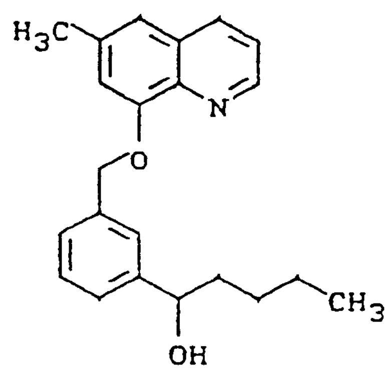

Ausbeute: 96% der Theorie
$R_f$ = 2.22 (System b)

Beispiel 102

Benzoesäure-1-[3-(chinolin-8-yloxymethyl)phenyl]pentylester

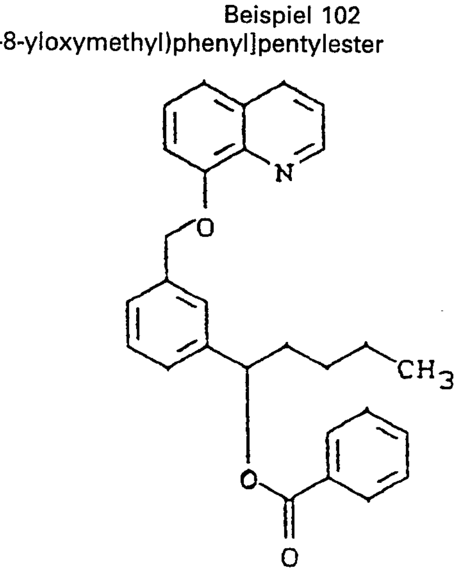

3.2 g (10 mmol) 1-[3-(Chinolin-8-yloxymethyl)phenyl]pentanol werden in 50 ml Dichlormethan gelöst.

Nach Zugabe von 2.8 ml Triethylamin und 1.16 ml Benzoylchlorid wird 2 h bei 25°C gerührt und anschließend 5 h unter Rückfluß erwärmt. Das Reaktionsgemisch wird dreimal mit Wasser gewaschen, über Natriumsulfat getrocknet und eingeengt. Der Rückstand wird über Keiselgel 60 chromatographiert (Laufmittel: Dichlormethan/Methanol 100:2). Man erhält ein Öl.

Ausbeute: 45% der Theorie

$R_f$ = 4.22 (System b)

Anwendungsbeispiel:

Beispiel 103

Die pharmakologische Wirkung der erfindungsgemäßen Substanzen wurde durch folgende Methode bestimmt:

Als Maß für eine Lipoxygenase-Hemmung wurde die Freisetzung von Leukotrien $B_4$ ($LTB_4$) an polymorphkernigen Rattenleukozyten (PMN) nach Zugabe von Substanzen und Calonophor mittels reverse phase HPLC nach Borgeat, P. et al., Proc. Nat. Acad. Sci., 76, 2148—2152 (1979) bestimmt. Die in vivo-Aktivität der Verbindungen wurde mit dem Mäuseohr-Entzündungsmodell nach Young, J.M. et al., J. of Investigative Dermatology, *82*, 367—371 (1984) nachgewiesen.

In Tabelle 1 sind beispielhaft die nach diesen Tets ermittelten Werte einiger erfindungsgemäßer Verbindungen aufgeführt:

| Bsp. Nr. | LO-Hemmung $IC_{50}$-Werte (g/ml) | Entzündungshemmung bei 2 mg/Ohr (%) |
|---|---|---|
| 38 | $1.4 \times 10^{-7}$ | 75.0 |
| 39 | $2.6 \times 10^{-7}$ | 72.0 |
| 55 | $8.3 \times 10^{-8}$ | 70.0 |
| 57 | $2.3 \times 10^{-7}$ | 86.1 |

**Patentansprüche**

1. Substituierte Benzylether der Formel

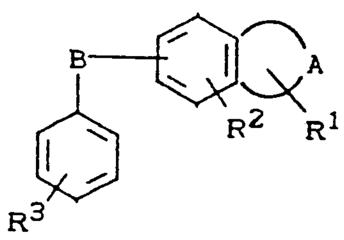

(I),

in welcher

$R^1$, $R^2$ gleich oder verschieden sind und für Wasserstoff, $C_1$ bis $C_8$-Alkyl, $C_2$ bis $C_6$-Alkenyl, Cyclopentyl, Cyclohexyl, $C_1$ bis $C_6$-Alkoxy, $C_1$ bis $C_6$-Alkylthio, Halogen $C_1$ bis $C_6$-Alkyl, Halogen $C_1$ bis $C_6$-Alkoxy Halogen $C_1$ bis $C_6$-Alkylthio, Benzyloxy, Benzylthio, Fluor, Chlor, Brom, Nitro, Cyano, Hydroxy oder für eine Gruppe der Formel

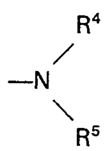

stehen,

wobei

$R^4$ und $R^5$ gleich oder verschieden sind und für Wasserstoff, $C_1$ bis $C_8$-Alkyl, $C_2$ bis $C_6$-Alkenyl, Cyclopentyl, Cyclohexyl, Benzyl, Phenyl, Benzoyl oder Acetyl stehen,

$R^3$ für eine Gruppe der Formel

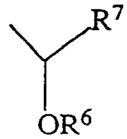 , 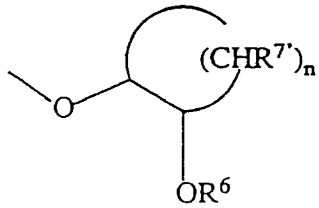 oder

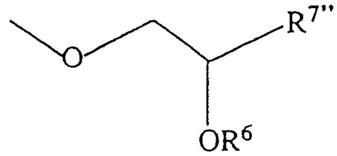 steht

worin

$R^6$ für Wasserstoff, Benzoyl, oder $C_1$ bis $C_6$-Alkyl-CO- steht,

$R^7$, $R^{7'}$,$R^{7''}$ gleich oder verschieden sind und für Wasserstoff oder $C_1$ bis $C_8$-Alkyl stehen und

n für eine Zahl von 3 bis 8 steht,

B für $—CH_2—X—$ steht, wobei

X für O, S oder $Nr^7$ steht und wobei

$R^7$ die oben genannte Bedeutung hat, und die Gruppe der Formel

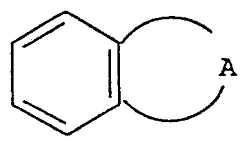

für Chinolin, Isochinolin, Cinnolin, Chinolin-N-oxid, Chinoxalin, Phthalazin, Chinolon, Isochinolon, Cinnolinon, Chinazolindion, Chinoxazolinon, Chinoxalindion, Phthalazindion, Indol, Indolon, Isoindol, Isoindolon, Benzofuran, Benzothiophen, Benzimidazol, Benzoxazol, Benzothiazol, Indazol, Indoxyl steht.

2. Substituierte Benzylether der Formel I nach Anspruch 1, worin

$R^1$, $R^2$ gleich oder verschieden sind und für Wasserstoff, Methyl, Ethyl, Propyl, Isopropyl, Allyl, Methoxy, Ethoxy, Methylthio, Ethylthio, Trifluormethylthio, Fluor, Chlor, Brom, Nitro, Cyano, Hydroxy oder für eine Gruppe der Formel

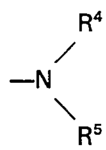 stehen,

wobei

$R^4$ und $R^5$ gleich oder verschieden sind und für Wasserstoff, Methyl, Ethyl, Propyl, Isopropyl, Allyl, Benzyl, Phenyl oder Acetyl stehen,

$R^3$ für einen Rest der Formel

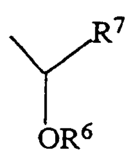 , 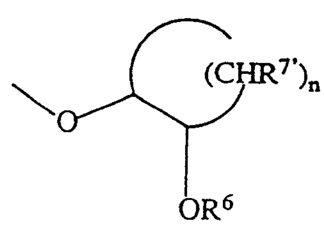 oder

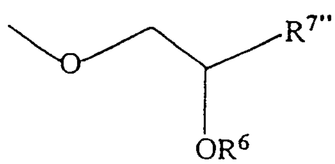 steht

wobei

$R^6$ für Wasserstoff, Benzoyl, Acetyl, Ethylcarbonyl oder Propylcarbonyl steht,

$R^7$, $R^{7'}$, $R^{7''}$ gleich oder verschieden sind und für Wasserstoff oder für eine gerade oder verzweigte Alkylkette mit bis zu 8 C-Atomen stehen und

n für eine Zahl von 3 bis 4 steht,

B für $—CH_2—X—$ steht, wobei

X für O, S oder $NR^7$ steht und wobei

$R^7$ die oben genannte Bedeutung hat, und die Gruppe der Formel

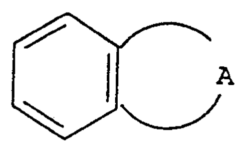

für Chinolin, Chinolin-N-oxid, Isochinolin, Isochinolin-N-oxid, Cinnolin, Chinazolin, Chinoxalin, Phthalazin, Chinolon, Isochinolon, Indol, Indolon, Isoindol, Benzofuran, Benzothiophen, Benzimidazol, Benzothiazol, Benzoxazol oder Indazol steht.

3. Substituierte Benzylether der Formel I nach Anspruch 1 worin

$R^1$, $R^2$ gleich oder verschieden sind und für Wasserstoff, Methyl, Ethyl, Fluor, Chlor, Brom und Nitro stehen,

$R^3$ für eine Gruppe der Formel

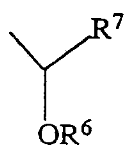 , 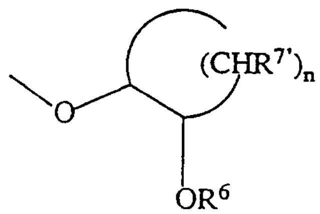 oder

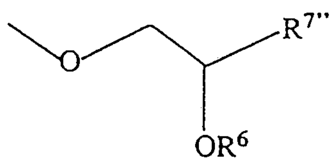 steht

worin

$R^6$ Wasserstoff, Acetyl, Ethylcarbonyl oder Benzoyl bedeutet, $R^7$, $R^{7'}$, $R^{7''}$ gleich oder verschieden sind und Wasserstoff oder eine geradkettige oder verzweigte Alkylkette mit bis zu 6 C-Atomen bedeutet,

n für eine Zahl 3 oder 4 steht,

B für $—CH_2—O—$, $—CH_2S—$ oder $—CH_2NH—$ steht, und die Gruppe der Formel

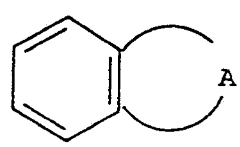

für Chinolin, Chinolin-N-oxid, Isochinolin, oder Chinolon steht.

4. Substituierte Benzylether nach den Ansprüchen 1 bis 3 zur therapeutischen Behandlung.

5. Verfharen zur Herstellung von substituierten Benzylethern der allgemeinen Formel

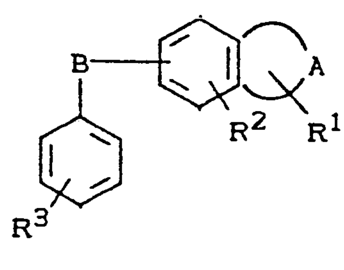 (I),

in welcher

$R^1$, $R^2$ gleich oder verschieden sind und für Wasserstoff, $C_1$ bis $C_8$-Alkyl, $C_2$ bis $C_6$-Alkenyl, Cyclopentyl,

Cyclohexyl, $C_1$ bis $C_6$-Alkoxy, $C_1$ bis $C_6$-Alkylthio, Halogen $C_1$ bis $C_6$-Alkyl, Halogen $C_1$ bis $C_6$-Alkoxy, Halogen $C_1$ bis $C_6$-Alkylthio, Benzyloxy, Benzylthio, Fluor, Chlor, Brom, Nitro, Cyano, Hydroxy oder für eine Gruppe der Formel

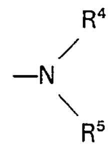 stehen,

wobei

$R^4$ und $R^5$ gleich oder verschieden sind und für Wasserstoff, $C_1$ bis $C_8$-Alkyl, $C_2$ bis $C_6$-Alkenyl, Cyclopentyl, Cyclohexyl, Benzyl, Phenyl, Benzoyl oder Acetyl stehen,

$R^3$ für eine Gruppe der Formel

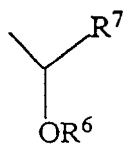 , 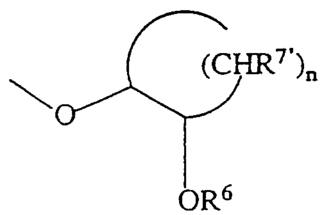 oder

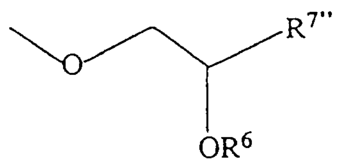 steht

worin

$R^6$ für Wasserstoff, Benzoyl, oder $C_1$ bis $C_6$-Alkyl-CO— steht, $R^7$, $R^{7'}$, $R^{7''}$ gleich oder verschieden sind und für Wasserstoff oder $C_1$ bis $C_8$-Alkyl stehen, und

n für eine Zahl von 3 bis 8 steht,

B für $—CH_2—X—$ steht, wobei

X für O, S oder $NR^7$ steht und wobei

$R^7$ die oben genannte Bedeutung hat, und die Gruppe der Formel

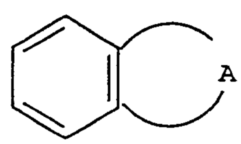

für Chinolin, Isochinolin, Cinnolin, Chinolin-N-oxid, Chinazolin, Isochinolin-N-oxid, Chinoxalin, Phthalazin, Chinolon, Isochinolon, Cinnolinon, Chinazolindion, Chinoxazolinon, Chinoxalindion, Phthalazindion, Indol, Indolon, Isoindol, Isoindolon, Benzofuran, Benzothiophen, Benzimidazol, Benzoxazol, Benzothiazol, Indazol, Indoxyl steht, dadurch gekennzeichnet, daß man Halogenide der allgemeinen Formel (II)

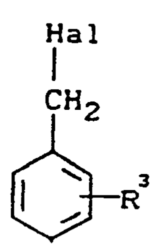 (II),

in welcher

$R^3$ für eine Gruppe der Formel

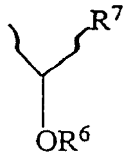 , 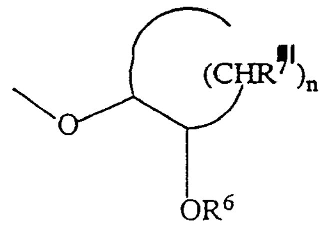 oder

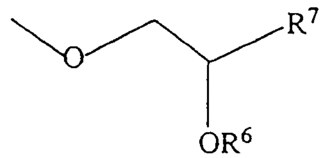 steht

wobei
$R^7$ und n die oben angegebene Bedeutung haben,
$R^6$ $C_1$ bis $C_6$-Alkyl-CO— oder Benzoyl bedeutet, und Hal für Chlor, Bromo oder Iod steht, mit Verbindungen der Formel

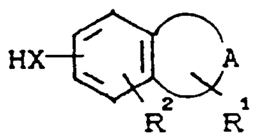 (III),

in welcher
$R^1$, $R^2$ und die Gruppe

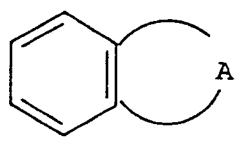

die oben angegebene Bedeutung haben, in inerten organischen Lösungsmitteln gegebenenfalls in Anwesenheit einer Base umsetzt, dann gegebenenfalls vorhandene Acylgruppen abspaltet.

6. Arzneimittel, enthaltend mindestens einen substituierten Benzylether nach Anspruch 1.
7. Arzneimittel nach Anspruch 6, enthaltend 0,5 bis 90 Gew.-% des substituierten Benzylethers.
8. Verwendung von substituierten Benzylethern nach Anspruch 1 zur Herstellung von Arzneimitteln.
9. Verwendung von substituierten Benzylethern zur Herstellung von Lipoxygenasehemmern.

**Revendications**

1. Ethers benzyliques substitués, de formule

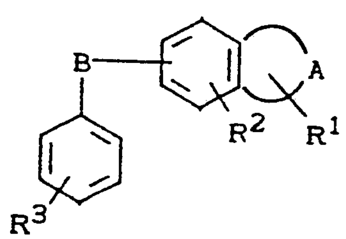 (I),

dans laquelle
$R^1$ et $R^2$ sont identiques ou différents et représentent chacun un atome d'hydrogène, un groupe alkyle en $C_1$ à $C_8$, alcényle en $C_2$ à $C_6$, cyclopentyle, cyclohexyle, alcoxy en $C_1$ à $C_6$, alkylthio en $C_1$ à $C_6$, halogéno-alkyle en $C_1$ à $C_6$, halogéno-alcoxy en $C_1$ à $C_6$, halogéno-alkylthio en $C_1$ à $C_6$, benzyloxy, benzylthio, un atome de fluor, de chlore ou de brome, un groupe nitro, cyano, hydroxy ou un groupe de formule

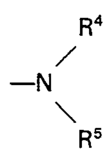

dans laquelle

$R^4$ et $R^5$ sont identiques ou différents et représentent chacun un atome d'hydrogène, un groupe alkyle en $C_1$ à $C_8$, alcényle en $C_2$ à $c_6$, cyclopentyle, cyclohexyle, benzyle, phényle, benzoyle ou acétyle,

$R^3$ représente un groupe de formules

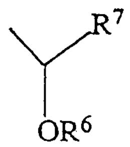

,

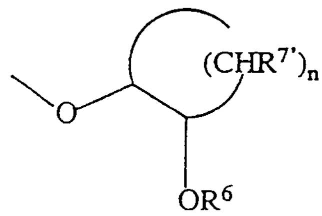

ou

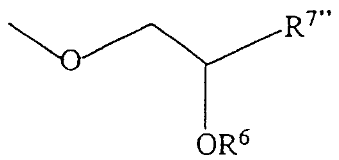

dans lesquelles

$R^6$ représente un atome d'hydrogène, un groupe benzoyle ou un groupe alkyl (en $C_1$ à $C_6$)—CO—,

$R^7$, $R^{7'}$, $R^{7''}$ sont identiques ou différents et représentent chacun un atome d'hydrogène ou un groupe alkyle en $C_1$ à $C_8$, et

n est un nombre valant 3 à 8,

B représente $—CH_2—X—$, où X représente O, S ou $NR^7$, et $R^7$ a le sens précité, et le groupe de formule

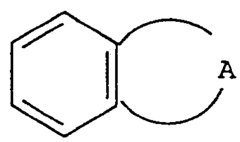

représente un groupe quinoléine, isoquinoléine, cinnoline, N-oxyde de quinoléine, quinoxaline, phtalazine, quinolone, isoquinolone, cinnolinone, quinazoline-dione, quinoxazolinone, quinoxaline-dione, phtalazine-dione, indole, indolone, isoindole, isoindolene, benzofuranne, benzothiophéne, benzimidazole, benzoxazole, benzothiazole, indazole, indoxyle.

2. Ethers benzyliques substitués de formule I selon la revendication 1, dans lesquels:

$R^1$ et $R^2$ sont identiques ou différents et représentent chacun un atome d'hydrogène, un groupe méthyle, éthyle, propyle, isopropyle, allyle, méthoxy, éthoxy, méthylthio, éthylthio, trifluorométhylthio, un atome de fluor, de chlore ou de brome, un groupe nitro, cyano, hydroxy ou un groupe de formule

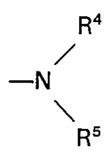

ou

$R^4$ et $R^5$ sont identiques ou différents et représentent chacun un atome d'hydrogène, un groupe méthyle, éthyle, propyle, isopropyle, allyle, benzyle, phényle ou acétyle,

$R^3$ représente un reste de formules

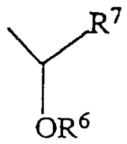
,
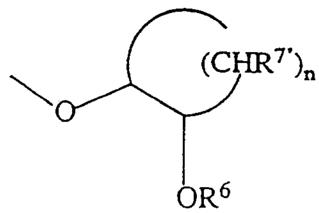
ou

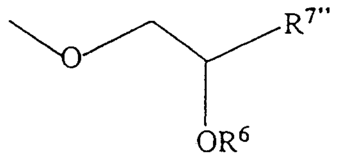

où

$R^6$ représente un atome d'hydrogène ou un groupe benzoyle, acétyle, éthylcarbonyle ou propylcarbonyle,

$R^7$, $R^{7'}$, $R^{7''}$ sont identiques ou différents et représentent chacun un atome d'hydrogène ou une chaîne alkyle, linéaire ou ramifiée, ayant jusqu'à 8 atomes de carbone, et

n est un nombre valant 3 à 4,

B représente un groupe $—CH_2—X—$, dans lequel

X représente O, S ou $NR^7$, et $R^7$ a le sens précité, et le groupe de formule

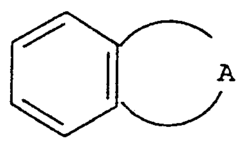

représente un groupe quinoléine, N-oxyde de quinoléine, isoquinoléine, N-oxyde d'isoquinoléine, cinnoline, quinazoline, quinoxaline, phtalazine, quinoline, isoquinolone, indole, indolone, isoindole, benzofuranne, benzothiophène, benzimidazole, benzothiazole, benzoxazole ou indazole.

3. Ethers benzyliques substitués de formule I selon la revendication 1, dans lesquels $R^1$ et $R^2$ sont identiques et différents et représentent chacun un atome d'hydrogène, un groupe méthyle ou éthyle, un atome de fluor, de chlore ou de brome ou un groupe nitro,

$R^3$ représente un groupe de formules

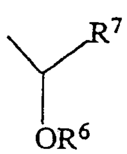
,
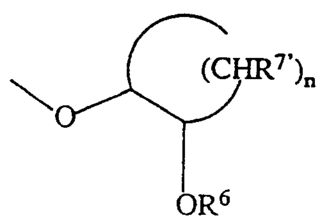
ou

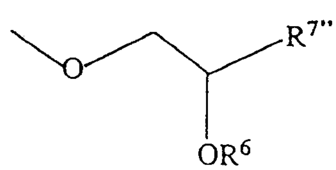

dans lesquelles

$R^6$ représente un atome d'hydrogène, un groupe acétyle, éthylcarbonyle ou benzoyle,

$R^7$, $R^{7'}$, $R^{7''}$ sont identiques ou différents et représentent chacun un atome d'hydrogène ou une chaîne, alkyle, linéaire ou ramifiée, ayant jusqu'à 6 atomes de carbone,

n est un nombre valant 3 ou 4,

B représente $—CH_2—O—$, $—CH_2S—$ ou $—CH_2NH—$,

et le groupe de formule

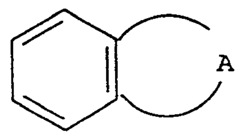

représente un groupe quinoléine, N-oxyde de quinoléine, isoquinoléine ou quinolone.

4. Ethers benzyliques substitués selon les revendications 1 à 3, pour un traitement thérapeutique.

5. Procédé pour préparer des éthers benzyliques substitués répondant à la formule générale

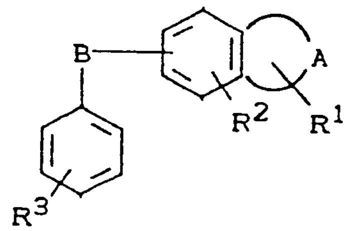

(I),

dans laquelle

$R^1$ et $R^2$ sont identiques ou différents et représentent chacun un atome d'hydrogène, un groupe alkyle en $C_1$ à $C_8$, alcényle en $C_2$ à $C_6$, cyclopentyle, cyclohexyle, alcoxy en $C_1$ à $C_6$, alkylthio en $C_1$ à $C_6$, halogénoalkyle en $C_1$ à $C_6$, halogénoalcoxy en $C_1$ à $C_6$, halogénoalkylthio en $C_1$ à $C_6$, benzyloxy, benzylthio, un atome de fluor, de chlore ou de brome, un groupe nitro, cyano, hydroxy ou un groupe de formule

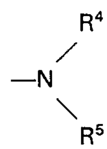

dans laquelle

$R^4$ et $R^5$ sont identiques ou différents et représentent chacun un atome d'hydrogène, un groupe alkyle en $C_1$ à $C_8$, alcényle en $C_2$ à $C_6$, cyclopentyle, cyclohexyle, benzyle, phényle, benzoyle ou acétyle,

$R^3$ représente un groupe de formules

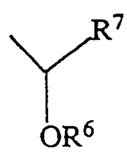

,

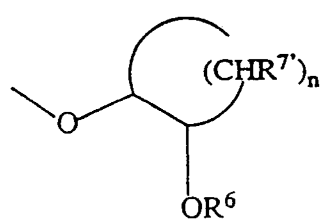

ou

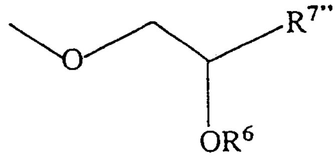

dans lesquelles

$R^6$ représente un atome d'hydrogène, un groupe benzoyle ou alkyle (en $C_1$ à $C_6$)—CO—,

$R^7$, $R^{7'}$, $R^{7''}$ sont identiques ou différents et représentent chacun un atome d'hydrogène ou un groupe alkyle en $C_1$ à $C_8$, et

n est un nombre valant 3 à 8,

B représente $—CH_2—X—$, groupe dans lequel X représente O, S ou $NR^7$, et $R^7$ a le sens précité, et le groupe de formule

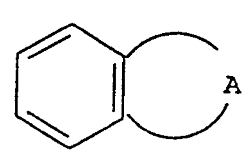

représente un groupe quinoléine, isoquinoléine, cinnoline, N-oxyde de quinoléine, quinazoline, N-oxyde

d'isoquinoléine, quinoxaline, phtalazine, quinolone, isoquinolone, cinnolinone, quinazoline-dione, quinoxazolinone, quinoxaline-dione, phtalazine-dione, indole, inodolone, isoindole, isoindolole, benzofuranne, benzothiophéne, benzimidazole, benzoxazole, benzothiazole, indazole, indoxyle, procédé caractérisé en ce qu'on fait réagir dans des solvants organiques inertes, éventuellement en présence d'une base, des halogénures de formule générale (II)

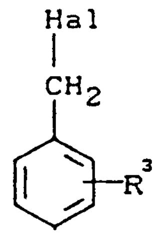

(II),

dans laquelle

$R^3$ représente un groupe de formules

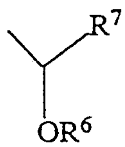

,

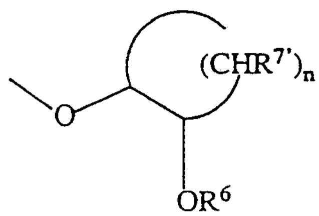

ou

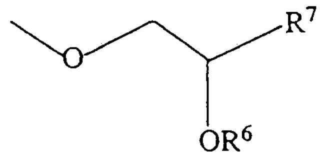

dans lesquelles

$R^7$ et n ont le sens précité,

$R^6$ représente un groupe alkyl (en $C_1$ à $C_6$)—CO— ou benzoyle, et Hal représente un atome de chlore, de brome ou d'iode, avec des composés de formule

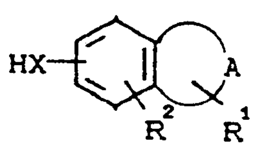

(III),

dans laquelle

$R^1$, $R^2$ et le groupe

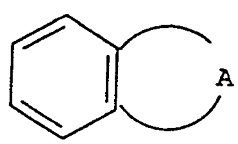

ont le sens précité, puis on scinde éventuellement les groupes acyles présents.

6. Médicaments contenant au moins un éther benzylique substitué selon la revendication 1.

7. Médicament selon la revendication 6, contenant 0,5 à 90% en poids de l'éther benzylique substitué.

8. Utilisation d'éthers benzyliques substitués selon la revendication 1, pour la préparation de médicaments.

9. Utilisation d'éthers benzyliques substitués pour préparer des inhibiteurs de la lipoxygénase.

**Claims**

1. Substituted benzyl ethers of the formula

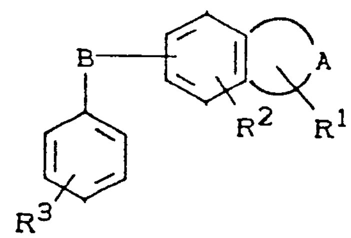

(I),

in which

$R^1$ and $R^2$ are identical or different and represent hydrogen, $C_1$ to $C_8$-alkyl, $C_2$ to $C_6$-alkenyl, cyclopentyl, cyclohexyl, $C_1$ to $C_6$-alkoxy, $C_1$ to $C_6$-alkylthio, halogeno-$C_1$ to $C_6$-alkyl, halogeno-$C_1$ to $C_6$-alkoxy, halogeno-$C_1$ to $C_6$-alkylthio, benzyloxy, benzylthio, fluorine, chlorine, bromine, nitro, cyano or hydroxyl, or represent a group of the formula

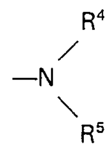

wherein

$R^4$ and $R^5$ are identical or different and represent hydrogen, $C_1$ to $C_8$-alkyl, $C_2$ to $C_6$-alkenyl, cyclopentyl, cyclohexyl, benzyl, phenyl, benzoyl or acetyl,

$R^3$ represents a group of the formula

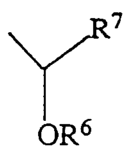

,

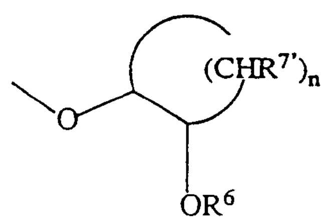

or

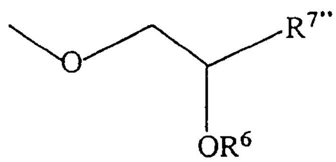

wherein

$R^5$ represents hydrogen, benzoyl or $C_1$ to $C_6$-alkyl—CO—,

$R^7$, $R^{7'}$ and $R^{7''}$ are identical or different and represent hydrogen or $C_1$ to $C_8$-alkyl, and n represents a number from 3 to 8, B represents —$CH_2$—X—,

wherein

X represents O, S or $NR^7$, and wherein

$R^7$ has the abovementioned meaning, and the group of the formula

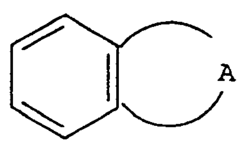

represents quinoline, isoquinoline, cinnoline, quinoline N-oxide, quinoxaline, phthalazine, quinolone, isoquinolone, cinnolinone, quinazolinedione, quinoxazolinone, quinoxalinedione, phthalazinedione, indole, indolone, isoindole, isoindolone, benzofuran, benzothiophene, benzimidazole, benzoxazole, benzothiazole, indazole or indoxyl.

2. Substituted benzyl ethers of the formula I according to Claim 1, wherein

$R^1$ and $R^2$ are identical or different and represent hydrogen, methyl, ethyl, propyl, isopropyl, allyl, methoxy, ethoxy, methylthio, ethylthio, trifluoromethylthio, fluorine, chlorine, bromine, nitro, cyano or hydroxyl, or represent a group of the formula

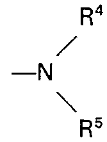

wherein

$R^4$ and $R^5$ are identical or different and represent hydrogen, methyl, ethyl, propyl, isopropyl, allyl, benzyl, phenyl, or acetyl,

$R^3$ represents a radical group of the formula

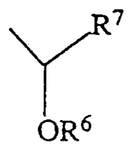 , 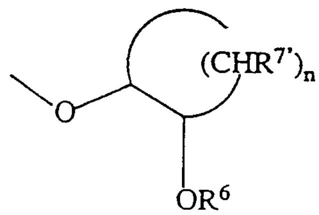 or

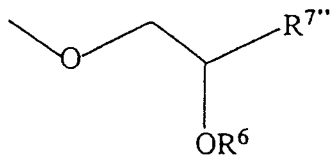

wherein

$R^5$ represents hydrogen, benzoyl, acetyl, ethyl-carbonyl or propylcarbonyl,

$R^7$, $R^{7'}$ and $R^{7''}$ are identical or different and represent hydrogen, or represent a straight or branched alkyl chain with up to 8 C atoms, and n represents a number from 3 to 4,

B represents $-CH_2-X-$,

wherein

X represents O, S or $NR^7$, and wherein

$R^7$ has the abovementioned meaning, and the group of the formula

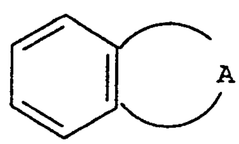

represents quinoline, quinoline N-oxide, isoquinoline, isoquinoline N-oxide, cinnoline, quinazoline, quinoxaline, phthalazine, quinolone, isoquinolone, indole, indolone, isoindole, benzofuran, benzothiophene, benzimidazole, benzothiazole, benzoxazole or indazole.

3. Substituted benzyl ethers of the formula I according to Claim 1, wherein

$R^1$ and $R^2$ are identical or different and represent hydrogen, methyl, ethyl, fluorine, chlorine, bromine and nitro,

$R^3$ represents a group of the formula

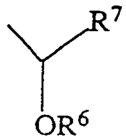 , 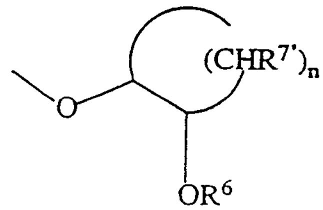 or

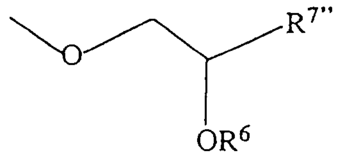

wherein

$R^6$ denotes hydrogen, acetyl, ethylcarbonyl or benzoyl,

$R^7$, $R^{7'}$ and $R^{7''}$ are identical or different and denote hydrogen or a straight-chain or branched alkyl chain with up to 6 C atoms,

n represents a number 3 or 4,

B represents $-CH_2-O-$, $-CH_2S-$ or $-CH_2NH-$, and the group of the formula

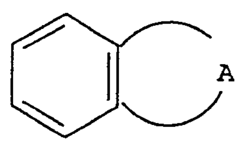

represents quinoline, quinoline N-oxide, isoquinoline or quinoline.

4. Substituted benzylethers according to Claims 1 to 3 for therapeutic treatment.

5. Process for the preparation of substituted benzyl ethers of the general formula

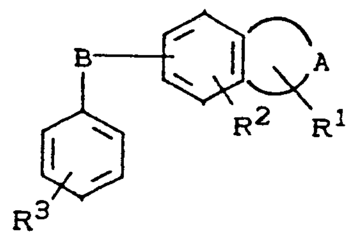 (I),

in which

$R^1$ and $R^2$ are identical or different and represent hydrogen, $C_1$ to $C_8$-alkyl, $C_2$ to $C_6$-alkenyl, cyclopentyl, cyclohexyl, $C_1$ to $C_6$-alkoxy, $C_1$ to $C_6$-alkylthio, halogeno-$C_1$ to $C_6$-alkyl, halogeno-$C_1$ to $C_6$-alkoxy, halogeno-$C_1$ to $C_6$-alkylthio, benzyloxy, benzylthio, fluorine, chlorine, bromine, nitro, cyano or hydroxyl, or represent a group of the formula

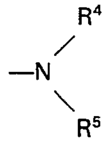

wherein

$R^4$ and $R^5$ are identical or different and represent hydrogen, $C_1$ to $C_8$-alkyl, $C_2$ to $C_6$-alkenyl, cyclopentyl, cyclohexyl, benzyl, phenzyl, benzoyl or acetyl,

$R^3$ represents a group of the formula

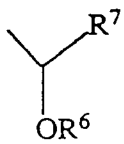 , 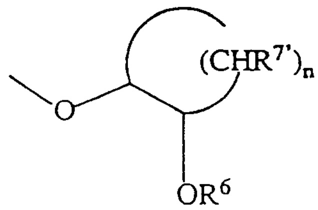 or

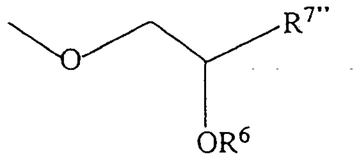

wherein

$R^6$ represents hydrogen, benzoyl or $C_1$ to $C_6$-alkyl-CO—,

$R^7$, $R^{7'}$ and $R^{7''}$ are identical or different and represent hydrogen or $C_1$ to $C_8$-alkyl, and

n represents a number from 3 to 8,

B represents $—CH_2—X—$,

wherein

X represents O, S or $NR^7$,

and wherein

$R^7$ has the abovementioned meaning, and the group of the formula

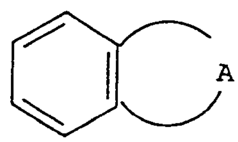

represents quinoline, isoquinoline, cinnoline, quinoline N-oxide, quinazoline, isoquinoline N-oxide, quinoxaline, phthalazine, quinolone, isoquinolone, cinnolinone, quinazolinedione, quinoxazolinone, quinoxalinedione, phthalazinedione, indole, indolone, isoindole, isoindolone, benzofuran, benzothiophene, benzimidazole, benzoxazole, benzothiazole, indazole or indoxyl, characterized in that halides of the general formula (II)

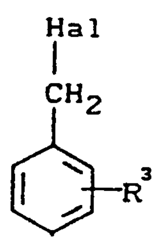 (II),

in which

$R^3$ represents a group of the formula

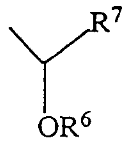 , 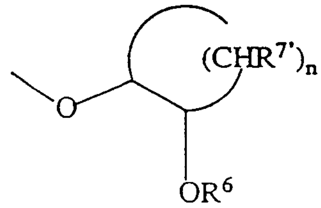 or

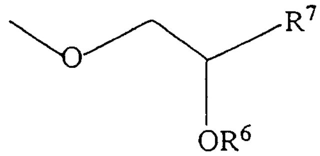

wherein

$R^7$ and n have the abovementioned meaning,

$R^6$ denotes $C_1$ to $C_6$-alkyl-CO— or benzoyl, and

Hal represents chlorine, bromine or iodine, are reacted with compounds of the formula

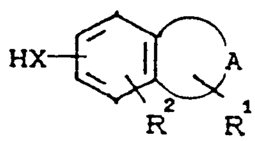

(III),

in which

$R^1$, $R^2$ and the group

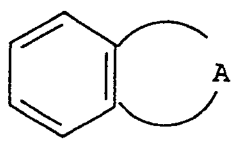

have the abovementioned meaning, are reacted in inert organic solvents, if appropriate in the presence of a base, and any acyl groups present are then split off.

6. Medicaments containing at least one substituted benzyl ether according to Claim 1.

7. Medicaments according to Claim 6, containing 0.5 to 90% by weight of the substituted benzyl ether.

8. Use of substituted benzyl ethers according to Claim 1 for the preparation of medicaments.

9. Use of substituted benzyl ethers for the preparation of lipoxygenase inhibitors.